(19) Europäisches Patentamt
European Patent Office
Office européen des brevets

(11) **EP 3 207 078 B1**

(12) # FASCICULE DE BREVET EUROPEEN

(45) Date de publication et mention de la délivrance du brevet:
**13.10.2021 Bulletin 2021/41**

(21) Numéro de dépôt: **15788172.3**

(22) Date de dépôt: **13.10.2015**

(51) Int Cl.:
***C08G 71/04*** *(2006.01)*
***C07C 271/12*** *(2006.01)*
***C08F 22/22*** *(2006.01)*
***C09D 4/00*** *(2006.01)*
***C09D 175/16*** *(2006.01)*
***C09J 175/16*** *(2006.01)*
***B33Y 10/00*** *(2015.01)*

(86) Numéro de dépôt international:
**PCT/FR2015/052754**

(87) Numéro de publication internationale:
**WO 2016/059340 (21.04.2016 Gazette 2016/16)**

(54) **OLIGOMERES URETHANE ACRYLATES MONO OU MULTIFONCTIONNELS SANS ISOCYANATE**

NON-ISOCYANAT MONO- ODER MULTI-ACRYLAT-URETHANOLIGOMERE

NON-ISOCYANATE URETHANE MONO- OR MULTIACRYLATE OLIGOMERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.10.2014 FR 1459890**

(43) Date de publication de la demande:
**23.08.2017 Bulletin 2017/34**

(73) Titulaire: **Arkema France**
**92700 Colombes (FR)**

(72) Inventeurs:
- **MONNIER, Guillaume**
  **F-60190 Avrigny (FR)**
- **LEROY, Catherine**
  **F-59000 Lille (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**US-A- 4 544 725**
**US-A1- 2004 192 803**
**US-A1- 2004 254 292**
**US-A1- 2013 004 677**

- **None**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 207 078 B1

## Description

L'invention concerne des nouveaux oligomères uréthanes monofonctionnels acrylates méthacrylates ou multifonctionnels en groupements acrylates et/ou méthacrylates, avec le groupement uréthane étant obtenu sans aucune utilisation d'isocyanate, par réaction carbonate-amine favorable à l'environnement et à la santé. L'invention utilise une voie NIPU qui correspond à « Non Isocyanate Polyuréthane », c'est-à-dire « polyuréthane sans isocyanate ». Elle permet l'obtention sélective des groupements acrylates et/ou méthacrylates avec une fonctionnalité minimale de 1, mais pouvant être élevée et avec en plus de cette fonctionnalité, une fonction hydroxyle par liaison uréthane créée. Ces oligomères peuvent être utilisés comme liants réticulables pour compositions de revêtements, d'adhésifs, de moulage ou d'agents d'étanchéité, de béton, de scellement chimique, de systèmes de fabrication d'objets en 3D couche par couche, de systèmes d'impression en 3D ou de béton ou de composite.

La synthèse actuelle d'oligomères uréthanes (méth)acrylates fait appel à l'utilisation de composés isocyanates ou poly-isocyanates pour les oligomères multifonctionnels. Ces composés sont toxiques et leur manipulation et utilisation demandent des précautions particulières et strictes pour préserver la santé humaine et pour l'environnement en général. De plus, la réaction d'uréthanisation (de formation de liaison uréthane par réaction d'alcool avec un isocyanate) utilise couramment des catalyseurs à base d'étain, composés également toxiques et nocifs pour l'environnement.

Ces dernières années ont vu un développement important de la réaction carbonate-amine comme voie alternative pour la formation d'une liaison uréthane sans utilisation d'isocyanates. Cette réaction permet en effet d'accéder à des composés uréthanes hydroxylés sans libération de composés secondaires lorsqu'elle fait intervenir un carbonate cyclique, c'est-à-dire un cycle carbonate à 5 ou 6 atomes avec hydroxyles en positions respectives en beta et en gamma du groupement uréthane. On obtient alors des composés dits « Non Isocyanate Polyuréthanes » ou en abrégé « NIPU». Les carbonates sont considérés comme peu nocifs ou dangereux en général. Il est d'autre part possible de préparer ces carbonates par addition de $CO_2$ sur une fonction époxy d'un composé époxydé. La préparation desdits carbonates par une telle voie consomme donc du $CO_2$, lequel est un gaz à effet de serre. Ainsi, sous réserve d'adopter ce mode de préparation, la substitution de la réaction alcool-isocyanate par la réaction carbonate-amine est bénéfique à la fois sur le plan de la sécurité et de l'environnement en général.

US 2013/0004677 A1 décrit un procédé de préparation de polyuréthane sans utiliser d'isocyanate par polymérisation par ouverture de cycle d'un composé bis-(carbonate cyclique) avec une diamine primaire. US 2004/254292 A1 et US 2004/192803 A1 décrivent un oligomère uréthane hydroxylé qui est le produit de réaction d'une diamine primaire et d'un composé carbonate cyclique.

L'objectif de la présente invention concerne des nouveaux oligomères uréthane acrylates ou méthacrylates de structure bien contrôlée et reproductible en termes de longueur de chaîne et de fonctionnalité, en particulier sans structure réticulée quand on fait réagir une polyamine porteuse de 2 à 5 groupements amines primaires et/ou secondaires sur un composé carbonate pouvant porter jusqu'à deux fonctions carbonates cycliques. Ces oligomères peuvent être monofonctionnels, aussi bien en acrylates qu'en méthacrylates, et/ou surtout des oligomères multifonctionnels en acrylates et/ou en méthacrylates et plus particulièrement de fonctionnalité d'au moins 3, qui sont convenables pour réticuler par voie radicalaire UV ou par peroxyde ou par voie duale, la voie duale étant une voie mixte utilisant un peroxyde par voie thermique et un photoamorceur par voie UV.

La présente invention permet en particulier d'accéder à de tels oligomères uréthanes acrylates ou méthacrylates, sans restriction particulière dans la manipulation ou le stockage des matières premières utilisées, non toxiques et amicales pour la santé humaine et l'environnement en général et disponibles commercialement.

La présente invention concerne plus particulièrement des oligomères porteurs de groupements uréthanes aminoacrylates et comme groupements réactifs finaux des acrylates et/ou méthacrylates et des hydroxyles en position beta ou gamma de ladite liaison uréthane en fonction du nombre d'atomes du cycle carbonate en respectivement 5 ou 6 atomes. Les groupements uréthanes et hydroxyles générés sont en nombre différent (inférieur) et en positionnement de la liaison uréthane indépendants des groupements acrylates ou méthacrylates sans liaison directe uréthane-ester acrylate ou méthacrylate. Il y a ainsi un effet multiplicateur sur la fonctionnalité finale de l'oligomère de l'invention en groupements acrylates et/ou méthacrylates, avec un contrôle de la structure et de la fonctionnalité desdits oligomères et sans risque de formation de gels lors de leur préparation, en particulier quand des oligomères multifonctionnels en acrylates et/ou méthacrylates sont visés. Ces oligomères présentent en particulier une vitesse de réticulation très satisfaisante sous UV et permettent l'obtention de produits d'application finis réticulés, en particulier des revêtements, ayant une bonne flexibilité et une dureté qui peut être ajustée en fonction du choix des réactants. Ainsi, les revêtements obtenus présentent en particulier un bon compromis de performances entre dureté et flexibilité tout en ayant les avantages de la présence des groupements uréthanes et aminoacrylates en plus. Ces avantages sont en particulier : résistance chimique, résistance aux solvants, résistance à l'abrasion et à l'usure et effet activateur synergiste des liaisons aminoacrylates lors de la photoréticulation.

L'autre avantage particulier de l'invention est qu'elle permet l'obtention d'oligomères polyuréthanes multiacrylates et/ou multiméthacrylates par un procédé plus simple et en seulement deux étapes par rapport à des étapes multiples souvent nécessaires dans les procédés habituels pour obtenir des oligomères uréthanes acrylés et/ou méthacrylés.

L'invention est telle que définie dans les revendications.

Le premier objet de l'invention concerne un oligomère uréthane monofonctionnel ou multifonctionnel en acrylates et/ou méthacrylates, obtenu à partir de la réaction d'une polyamine spécifique a) avec un composé carbonate cyclique b), ladite polyamine étant en excès stœchiométrique et donnant un produit intermédiaire c) porteur de groupements résiduels réactifs amine et dans une deuxième étape, addition de chacun desdits groupements résiduels réactifs amine -NH- sur un groupement acrylate d'un composé d) porteur en plus dudit groupement acrylate, de groupements supplémentaires acrylates ou méthacrylates, avec obtention dudit oligomère uréthane selon l'invention.

La présente invention concerne également un procédé de préparation dudit oligomère comportant deux étapes i) réaction de ladite polyamine a) sur ledit carbonate b) donnant ledit produit intermédiaire c) et ii) réaction de modification dudit produit c) par réaction d'addition de Michael de chacun desdits groupements résiduels réactifs amine -NH- sur un groupement acrylate dudit composé d).

Un autre objet de l'invention concerne une composition réticulable comprenant au moins un oligomère tel que défini selon la présente invention.

La présente invention couvre également l'utilisation dudit oligomère comme liant réactif dans des compositions de revêtements incluant peintures, vernis, encres, compositions d'adhésifs, compositions de moulage, compositions d'agent d'étanchéité, compositions de scellement chimique, compositions de systèmes de fabrication d'objets en 3D couche par couche, compositions de systèmes d'impression en 3D, compositions de béton ou compositions de composite.

Finalement, la présente invention couvre le produit fini qui résulte de l'utilisation d'au moins un oligomère tel que défini selon l'invention ou un oligomère tel qu'obtenu par le procédé de l'invention, ce produit fini étant de préférence choisi parmi : les revêtements incluant les peintures, les vernis et les encres, les adhésifs, les pièces moulées, les objets en 3D obtenus couche par couche, les objets d'impressions en 3D, les joints d'étanchéité, le scellement chimique, le béton fini ou les articles en composite.

Le premier objet de l'invention concerne donc un oligomère uréthane monofonctionnel ou multifonctionnel en acrylates et/ou méthacrylates, de préférence acrylates, lequel est susceptible d'être obtenu à partir de la réaction d'une polyamine spécifique a) avec un composé carbonate cyclique b) porteur de m groupements carbonates cycliques, ladite polyamine étant en excès stœchiométrique par rapport à b) et ladite réaction donnant un produit intermédiaire c) porteur de m groupements uréthanes formés, porteurs de groupements résiduels réactifs amine -NH- et ensuite par une réaction d'addition de chacun desdits groupements résiduels réactifs amine dudit produit c) sur un groupement acrylate d'un composé d) porteur en plus dudit groupement acrylate, de p groupements supplémentaires acrylates et/ou méthacrylates, avec chaque groupement résiduel réactif amine -NH- dudit produit c) étant ainsi transformé en liaison carbone-azote porteuse desdits groupements acrylates et/ou méthacrylates et obtention ainsi dudit oligomère uréthane, avec

- ladite polyamine a) étant porteuse de n groupements amine primaire et/ou secondaire et en option d'au moins un groupement amine tertiaire, avec n allant de 2 à 5, de préférence de 2 à 4 et plus préférentiellement de 2 à 3, encore plus préférentiellement de 2,
- ledit carbonate b) étant porteur de m groupements carbonates cycliques avec m allant de 1 à 2, de préférence le cycle desdits groupements carbonates cycliques étant un cycle à 5 ou à 6 atomes,
- ledit produit intermédiaire c) étant porteur de m groupements uréthanes et de m groupements OH en alpha ou beta dudit groupement uréthane et c) étant porteur de m*(n-1) à m*(2n-2) groupements résiduels réactifs amine -NH-,
- ledit composé d) étant porteur de p groupements acrylates et/ou méthacrylates en plus dudit groupement acrylate qui réagit avec un desdits groupements résiduels réactifs amine -NH- avec p étant au moins égal à 1 et de préférence au moins égal à 2 et
- le nombre desdits groupements résiduels réactifs amine -NH- dudit produit c) étant inférieur ou égal au nombre de moles dudit composé d)
- chaque liaison carbone-azote formée étant porteuse de p groupements acrylates et/ou méthacrylates et ledit oligomère uréthane étant porteur de m groupements uréthanes et de m groupement hydroxyles en alpha ou beta dudit uréthane et ayant une fonctionnalité en acrylates et/ou méthacrylates allant de m*p(n-1) à m*p(2n-2).

Lesdits n groupements amine primaire et/ou secondaire de ladite polyamine a) peuvent être portés par un radical A de valence n, lequel radical A peut être ou comprendre une structure aliphatique, cycloaliphatique ou aromatique avec dans ce dernier cas lesdits n groupements amines étant portés par au moins un groupement alkylène attaché au noyau aromatique et les cycles desdits m groupements carbonates cycliques dudit carbonate b) sont à 5 ou 6 atomes et portés par un radical R3 de valence m, avec R3 étant un radical hydrocarboné de structure aliphatique, cycloaliphatique ou aromatique et en option hydroxylé ou R3 peut être un simple hydrogène pour m = 1, en option le cycle carbonate peut être substitué par un substituant $R_4$ hydrocarboné éventuellement hydroxylé, $R_4$ pouvant être de structure aliphatique, cycloaliphatique ou aromatique identique ou différente de celle de $R_3$ et éventuellement pouvant former un cycle attaché au cycle carbonate par deux atomes de carbone communs.

Le terme « réactif » concernant lesdits groupements résiduels réactifs amine -NH- signifie selon la présente invention « pouvant réagir par addition de Michael sur une fonction acrylate avec formation d'un groupement aminoacrylate —N-CH2-CH2-$CO_2$- ».

Sur la base dudit radical A, ladite polyamine a) est globalement représentée par : $(R'_1\text{-NH-})_xA(\text{-NH2})_y$, avec $R'_1$ étant un alkyl ou cycloalkyle ou aralkyle et avec x + y = n.

La structure cycloaliphatique telle que citée ci-haut comprend les structures polycycliques comme les bicycles ou tricycles aliphatiques.

Plus particulièrement, ladite polyamine a) peut être de structure aliphatique en $C_2$ à $C_{54}$ ou de structure cycloaliphatique y compris polycyclique comme bicycle ou tricycle ou elle peut comprendre une structure aromatique avec les fonctions amines portées par des alkylènes attachés au noyau aromatique.

Ladite polyamine a) peut être de structure monomérique et donc sans unité répétitive ou oligomérique avec au moins deux unités monomériques répétitives liées à un groupement amine, par exemple alkylène imine comme unité répétitive, en particulier l'éthylène imine ou à un autre groupement tel qu'un éther, ester ou amide.

Selon une option particulière liée à la structure du radical A de ladite polyamine a) porteur desdits n groupements amines primaires et/ou secondaires, ladite polyamine a) est choisie parmi :

- une alkylène polyamine en $C_2$ à $C_{54}$, ledit alkylène étant en $C_2$ à $C_{54}$ linéaire ou ramifié et porteur desdits n groupements amine primaire et/ou secondaire,
- une polyamine comprenant au moins deux unité répétitives parmi : alkylène imine en $C_2$ à $C_4$, en particulier éthylène imine ou propylène imine, de préférence éthylène imine, ladite polyamine a) étant dans ce cas une oligoalkylène imine polyamine, en particulier une polyéthylène imine polyamine y compris de structure dendritique,
- une polyamine comprenant une ou plusieurs unités parmi : éther, ester-amide, amide, avec dans le cas de présence de plusieurs unités, ladite polyamine a) étant choisi parmi : oligoéthers polyamines, oligoester amide-polyamines ou multiester amide-polyamines.

Parmi les polyamines a) en $C_2$ à $C_{54}$, on peut citer les diamines ou triamines à chaîne aliphatique linéaire parmi les alkylènes diamines ou alkylènes triamines, y compris les diamines grasses en $C_{36}$ ou ou triamines grasses en $C_{54}$, avec lesdits alkylènes pouvant être alcoxylés ou non alcoxylés.

Parmi les polyamines, une ou plusieurs unités parmi : éther, ester-amide, amide, on peut citer dans le cas de plusieurs unités, les polyéthylènes imines polyamines de préférence oligoéthylène imine polyamines, les oligoéther polyamines telles que les Jeffamines R, les oligoester-amide polyamines ou les multi (ester-amide amines) qui sont des esters multifonctionnels terminés (chaque ester) par un groupement amide-amine.

Concernant ladite fonctionnalité globale f dudit oligomère uréthane selon l'invention, elle peut avoir la valeur minimale quand tous les groupements amines de la polyamine a) sont tous secondaires et la valeur maximale quand tous les groupements amines de la polyamine a) sont primaires. Par conséquent, cette fonctionnalité f ne peut que varier entre les deux limites telles que définies.

Ladite polyamine a) est choisie parmi les polyamines de formules a1) ou a2) suivantes :

a1) $R_1\text{-NH-}R_2\text{-(NH-R)(NH-R')}_{n1}$ avec $n_1$ étant égal à 3, 2, 1 ou 0 et de préférence $n_1$ étant égal à 2, 1 ou 0

a2) $R_1\text{-NH-}(R'_2\text{NH})_{n2}\text{-}R''_2\text{-NH-R'}$ avec $n_2$ étant égal à 3, 2 ou 1

avec $R_1$ et R et R' étant choisis indépendamment parmi : H, alkyle, de préférence $C_1$-$C_3$, en option ledit alkyle étant alcoxylé ou un cyclolalkyle, avec $R_1$ pouvant être identique ou différent de R et R' et avec R et R' pouvant être identiques ou différents et en particulier avec R et/ou R' étant différents de H,

$R_2$ étant alkylène ou cycloalkylène (comprenant un cycle aliphatique en $C_6$) ou aralkylène de valence n allant de 2 à 5, avec $R_2$ pouvant porter ou comprendre au moins un groupement amine tertiaire

et

$R'_2$ et $R''_2$ pouvant être identiques ou différents et choisis parmi alkylènes, de préférence en $C_2$ à $C_8$, plus préférentiellement en $C_2$ à $C_6$ et si $R''_2$ est différent de $R'_2$ dans ce cas $R''_2$ pouvant porter ou comprendre au moins un groupement amine tertiaire.

Selon une réalisation particulière dudit oligomère selon l'invention, ladite polyamine a) est une amine selon formule a2) définie ci-dessus, avec $n_2$ = 1 et $R_1$ et R' étant H et $R'_2$ et $R''_2$ étant des alkylènes identiques ou différents et en $C_2$ à $C_6$, en particulier en $C_2$ à $C_4$, plus particulièrement ladite amine a2) étant sélectionnée parmi 3(2-aminoéthyl)aminopropylamine ou bis(3-aminopropyl)amine.

Selon un choix particulier, ladite polyamine a) est une amine définie selon formule a2), comme décrite ci-haut avec $n_2$ = 2 ou 3 et $R_1$ et R' étant H et $R'_2$ et $R''_2$ étant des alkylènes identiques en $C_2$ à $C_4$, en particulier en $C_2$ à $C_3$,

plus particulièrement ladite amine a2) étant sélectionnée parmi triéthylènetetramine ou tetraéthylènepentamine.

Plus particulièrement, ladite polyamine est une diamine (n = 2) et de préférence sélectionnée parmi les diamines secondaire-primaires, plus préférentiellement parmi : N-méthyl 1,3-propane diamine, N-méthyl éthane diamine, N-méthyl 1,4- butane diamine, N-méthyl 1,5- pentane diamine.

Selon une seconde option particulièrement préférée, ladite polyamine a) est une diamine (n = 2) et elle est secondaire-secondaire, plus préférentiellement parmi : N-méthyl, N'méthyl 1,3-propane diamine, N-méthyl, N'méthyl éthane diamine, N-méthyl, N'méthyl 1,4- butane diamine, N-méthyl, N'méthyl 1,5- pentane diamine.

Le composé carbonate b) selon l'invention peut être représenté globalement par la formule globale $R_3$(-cyclecarbo)$_m$ avec m allant de 1 à 2 et « cyclecarbo » étant ledit groupement carbonate cyclique.

Ledit composé b) peut être un mélange de composés carbonate porteurs de m = 1 et/ou m = 2 carbonates cycliques. Quand m est entre 1 et 2, il s'agit d'un mélange d'au moins un monocarbonate b) avec m = 1 et d'au moins un dicarbonate b) avec m = 2.

La polyamine a) peut être également un mélange de différentes polyamines a) de nature différente et/ou avec n différent ou identique.

Les composés d) peuvent également être un mélange d'au moins deux composés d) selon l'invention de nature différente avec une fonctionnalité différente ou identique.

La définition dudit radical hydrocarboné $R_3$ porteur de m groupements carbonates cycliques dans ledit composé carbonate cyclique b) dépend de la structure dudit carbonate b). En particulier, ledit carbonate b) est un ester complet ou partiel d'un polyacide, monomérique ou oligomérique tel qu'un oligoester polyacide, avec un carbonate hydroxylé, de préférence le carbonate de glycérol ou b) est un éther complet ou partiel d'un polyol monomérique ou oligomérique tel que oligoester ou oligoéther polyol avec un carbonate hydroxylé, de préférence le carbonate de gycérol ou b) est un éther complet ou partiel d'un dérivé phénolique, y compris bisphénol A ou dihydroxy-phénylène avec un carbonate hydroxylé, de préférence le carbonate de glycérol ou b) est un éther d'alcool allylique ou vinylique avec un carbonate hydroxylé, de préférence le carbonate de glycérol ou un méthacrylate d'un carbonate hydroxylé, de préférence du carbonate de glycérol ou b) est un carbonate obtenu par addition de $CO_2$ sur un composé précurseur monomérique ou oligomérique époxydé ou b) est le produit de la réaction du diméthylcarbonate avec un polyol aliphatique ou cycloaliphatique de fonctionnalité d'au moins 2, de préférence allant de 2 à 6.

Plus particulièrement, ledit précurseur époxydé peut être un oligodiène époxydé, un ester méthylique ou éthylique d'un acide gras insaturé époxydé ou un dérivé époxydé d'un alcool gras insaturé (chaîne grasse mono ou diinsaturée) ou un prépolymère époxydé obtenu par réaction amine-époxyde, prépolymère polyester époxydé par réaction anhydride-époxy, oligomère acrylique époxydé et/ou oligomère styrénique époxydé ou un éther de glycidyle alcool avec un polyol ou un ester de glycidyle alcool avec un polyacide, avec ledit polyol ou polyacide pouvant être monomérique ou oligomérique ou une oléfine époxydée (pour m = 1) ou une dioléfine époxydée.

Ainsi, quand ledit précurseur époxydé est un oligodiène époxydé ou un ester d'acide gras insaturé époxydé ou un dérivé d'alcool gras insaturé époxydé, dans ce cas, ledit carbonate b) peut être une chaîne aliphatique mono ou multifonctionnelle en carbonates cycliques avec 2 atomes de carbone du cycle carbonate dudit composé carbonate b) faisant partie intégrale de ladite chaîne.

Comme déjà décrit, ledit carbonate b) peut être également obtenu par la réaction du diméthylcarbonate avec un polyol aliphatique ou cycloaliphatique de fonctionnalité d'au moins 2, de préférence allant de 2 à 6.

Selon une option particulière, ledit cycle carbonate est substitué par ledit substituant $R_4$ comme défini ci-haut, lequel est un alkyle éventuellement hydroxylé et en option plus particulière $R_4$ peut former un cycle attaché au cycle carbonate par 2 atomes de carbone communs.

Selon une variante préférée de l'invention, le cycle du carbonate cyclique b) est un cycle à 5 atomes.

Les composés carbonates b peuvent être aussi obtenus à partir d'un précurseur mono ou polyépoxydé, de préférence polyépoxydé, porteur de m groupements époxy, modifiés en carbonates cycliques par addition de dioxyde de carbone sur lesdits groupements époxy.

Comme oligodiène époxydé, on peut citer un oligobutadiène époxydé ou un polyisoprène époxydé ou des copolymères de butadiène et d'isoprène époxydés oligomériques. Comme dioléfine ou oléfine époxydée, on peut citer le dicyclopentadiène époxydé, le norbornène époxydé, le cyclohexène époxydé, le cyclohexadiène époxydé qui sont des carbonates b) de structure cycloaliphatique. Comme composé époxydé précurseur d'un carbonate convenable comme carbonate cyclique b) selon l'invention, on peut citer également les prépolymères époxydés obtenus avec excès d'époxyde par réaction amine-époxyde ou prépolymère polyester époxydé par réaction anhydride-époxy, oligomère acrylique et/ou styrénique époxydé, en particulier copolymère acrylique et/ou styrénique de glycidyl méthacrylate ou un monomère polyépoxydé qui est un éther ou un ester de glycidyle, en particulier les dérivés glycidyl éthers des bisphénols, de préférence hydrogénés ou les glycidyl éthers des polyols aliphatiques ou des polyols cycloaliphatiques ou des glycidyl esters de polyacides de fonctionnalité initiale m en carboxy avant estérification.

Comme composé précurseur polyépoxydé, peut convenir tout époxyde de structure monomérique ou de structure oligomérique linéaire ou ramifiée, avec dans le cas d'oligomère, de préférence ayant une masse moléculaire en nombre Mn inférieure à 1000 et de préférence inférieure à 600 Daltons et mesurée par GPC en équivalents polystyrène.

Plus particulièrement, le composé époxydé précurseur dudit carbonate b) selon l'invention peut être un monomère diépoxyde et peut être choisi parmi : diglycidyl éther d'éthylène glycol, diglycidyl éther de propylène glycol, diglycidyl éther de butanediol, diglycidyl éther de néopentyl glycol, diglycidyl éther d'hexanediol, diglycidyl éther de cyclohéxane diméthanol, diglycidyl éther de bisphénol A (DGEBA) hydrogéné ou non hydrogéné, diglycidyl éther de bisphénol F (DGEBF) hydrogéné ou non hydrogéné, ces composés étant éventuellement alcoxylés, par exemple avec 1 à 4 unités alcoxy comme éthoxy et/ou propoxy ou diglycidyl ester d'acide ortho- iso- ou téréphtalique, diglycidyl ester d'acide tetrahydrophtalique ou diglycidyl ester d'acide hexahydrophtalique.

Ledit composé mono ou polyépoxydé, en particulier diépoxydé, peut être également un oligomère époxydé ou une résine époxydée comme les huiles époxydées, en particulier de soja, du polybutadiène époxydé ou un prépolymère de condensation ou d'addition portant des groupements terminaux époxydes (ou époxy ou oxiranes). La fonctionnalité en époxy de ce composé époxydé doit correspondre à la fonctionnalité m en carbonates cycliques dudit carbonate b) visé. Par exemple, pour un dicarbonate, c'est-à dire ayant 2 carbonates cycliques, le précurseur époxydé est de préférence un diépoxyde appelé aussi diépoxy.

Ledit carbonate b) pour m = 2 peut également être en alternative le produit de la réaction du diméthylcarbonate avec un polyol aliphatique ou cycloaliphatique de fonctionnalité d'au moins 2, de préférence allant de 2 à 6, avec formation desdits composés carbonates cycliques b) tels que définis selon l'invention.

Ledit composé d) est un acrylate multifonctionnel, dans la mesure où il porte au moins un groupement acrylate, un acrylate étant le minimum si l'autre groupement est un méthacrylate et au moins deux acrylates en l'absence de tout groupement méthacrylate avec un minimum de deux acrylates dans ce cas. Ce minimum de fonctionnalité de départ dudit composé d) est celle requise quand on vise un oligomère uréthane monofonctionnel en acrylate ou méthacrylate en partant d'un composé carbonate b) monofonctionnel et d'une polyamine a) bifonctionnelle (n = 2).

Il est à noter que la réaction entre un groupement amine, qu'il soit primaire ou secondaire, et un groupement carbonate donne toujours un groupement uréthane, avec la seule différence, dans le cas d'un groupement d'amine secondaire par rapport à l'amine primaire, que l'atome d'azote du groupement uréthane est substitué (si amine secondaire) au lieu de porter un hydrogène (si amine primaire).

Par contre, les deux groupements résiduels -NH- d'un groupement d'amine primaire après la formation des m groupements uréthanes peuvent réagir avec deux groupements acrylates sur deux molécules différentes de composé d).

Ledit composé d) peut être représenté globalement par

$$R_5\text{-}(O_2CCH{=}CH_2)(\text{-}OCC(R_6){=}CH_2)_p$$

avec $R_5$ étant un résidu hydrocarboné de valence p+1 et $R_6$ est H ou méthyl pour une partie ou la totalité de p groupements et de préférence $R_5$ est un résidu de polyol choisi parmi alkylène polyol en option alkoxylé et/ou substitué, cycloalkylène polyol en option substitué ou aralkylène polyol en option alcoxylé et/ou substitué sur le cycle aromatique ou polyéther polyol ou polyester polyol ou $R_5$ est un résidu d'un composé époxy acrylate multifonctionnel porteur d'un groupement acrylate et de p groupements supplémentaires acrylates et/ou méthacrylates.

Ledit composé d) est selon une première option un monomère acrylique multifonctionnel ayant une fonctionnalité d'au moins 2, en particulier supérieure à 2 et allant jusqu'à p+1 égal à 6 et sélectionné parmi :

d1) les esters acrylates de polyols alcoxylés ou non alcoxylés, de préférence polyols choisis dans le groupe : trimethylol propane, ditrimethylol propane, pentaérythritol, dipentaérythritol, diéthylène glycol, dipropylène glycol, tripropylène glycol, glycérol, propylène glycol, butane diol, hexane diol ou
d2) les époxy acrylates,
d3) les aminoacrylates.

Selon une deuxième option, ledit composé d) est un oligomère acrylique multifonctionnel ayant une fonctionnalité d'au moins 2, en particulier supérieure à 2 et allant jusqu'à p+1 égal à 12 et sélectionné parmi :

d4) oligomère acrylique acrylé qui peut être un copolymère de glycidyl méthacrylate acrylé ou oligomère styrénique acrylé qui peut être un copolymère de styrène avec l'anhydride maléique ou l'acide acrylique qui est acrylé par l'hydroxy éthyl acrylate,
d5) oligomère hydroxylé acrylé, en particulier parmi polydiènes hydroxylés acrylés, de préférence le polybutadiène hydroxylé acrylé et plus préférentiellement le polybutadiène hydroxylé hydrogéné acrylé,
d6) huile époxydé acrylée,
d7) oligodiène époxydé acrylé,
d8) oligoéthers acrylates,
d9) oligoesters acrylates,

d10) oligoaminoacrylates.

Le terme « acrylates » selon ces options ci-dessous signifie que le composé d) peut porter uniquement des groupements acrylates $-O_2C-CH=CH_2$ ou bien il peut porter au moins un acrylate et p groupements dont une partie ou la totalité sont des groupements méthacrylates selon $-O_2C-C(CH_3)=CH_2$. Selon une option particulière, d) porte uniquement des groupements acrylates (sans méthacrylate), ce qui donne une réactivité plus élevée à l'oligomère correspondant selon l'invention.

Selon une préférence particulière dudit oligomère selon l'invention, ladite polyamine a) est une diamine. Plus précisément, ladite polyamine a) peut être une diamine sélectionnée parmi les diamines secondaire-primaire, primaire-primaire ou secondaire-secondaire, de préférence secondaire-primaire ou secondaire-secondaire.

Selon une option plus particulière, ladite diamine est sélectionnée parmi les diamines secondaire-primaire, de préférence parmi : N-méthyl 1,3-propane diamine, N-méthyl éthane diamine, N-méthyl 1,4-butane diamine, N-méthyl 1,5- pentane diamine.

Selon une autre option plus particulière, ladite diamine est sélectionnée parmi les diamines secondaire-secondaire, de préférence parmi : N-méthyl N'méthyl 1,3-propane diamine, N-méthyl N'méthyl éthane diamine, N-méthyl, N'méthyl 1,4- butane diamine, N-méthyl, N'méthyl 1,5- pentane diamine.

Selon une option particulièrement préférée de l'oligomère de l'invention, le cycle carbonate dudit carbonate b) est à 5 atomes, ladite polyamine a) est une diamine secondaire-primaire avec $n_1 = 0$ et R = H comme définie selon formule a1) telle que définie ci-haut et ledit oligomère comprend au moins un oligomère de formule générale (I) suivante :

$$[(CH_2=CH(R_6)-CO_2)_p-R_5-O_2C-CH_2-CH_2-N(R_1)-R_2-NH-CO_2-C(R_4)-CH(OH)-]_mR_3 \quad (I)$$

avec

- $R_1$ étant tel que défini ci-haut dans la formule a1)
- $R_2$ étant tel que défini ci-haut dans la formule a1)
- $R_3$ étant tel que défini pour le composé b) le radical de valence m, porteur desdits m groupements carbonates cycliques
- $R_4$ étant ledit substituant optionnel du cycle carbonate comme défini ci-haut
- $R_5$ étant tel que défini pour ledit composé d) ci-haut
- $R_6$ étant H ou méthyle ou H et méthyle si p est différent de 1.

Selon une autre option, ledit cycle carbonate dudit carbonate b) est à 6 atomes, ladite polyamine a) est une diamine secondaire-primaire avec $n_1 = 0$ et R = H comme définie selon formule a1) ci-haut et ledit oligomère comprend au moins un oligomère de formule générale (II) suivante :

$$[(CH_2=CH(R_6)-CO_2)_p-R_5-O_2C-CH_2-CH_2-N(R_1)-R_2-NH-CO_2-C(R_4)-CH_2CH(OH)-]_mR_3 \quad (II)$$

avec $R_1$, $R_2$, $R_3$, $R_4$, $R_5$ et $R_6$ étant tels que définis ci-haut dans formule (I).

Plus particulièrement, la fonctionnalité f en groupements acrylates et/ou méthacrylates dudit oligomère uréthane selon la présente invention peut varier de 1 à 30, de préférence de 2 à 20, plus particulièrement de 3 à 20.

Selon un cas particulier et préféré d'oligomère comme défini selon l'invention, p varie de 1 à 11, de préférence de 2 à 11, plus préférentiellement de 2 à 5, encore plus préférentiellement étant supérieur à 2 et allant jusqu'à 5.

Selon un cas plus particulier pour cet oligomère de l'invention, m = 2 et p varie de 2 à 11 et de préférence p varie de 3 à 5.

Ledit oligomère est de structure linéaire ou ramifiée. Par définition, il ne peut comporter de structure réticulée, laquelle est ainsi exclue. L'homme du métier sait en particulier choisir les proportions et les fonctionnalités de composants réactifs ainsi que le taux de conversion des fonctions réactives afin d'éviter toute réticulation ou gélification chimique du système réactif. Cette question ne peut se poser que quand l'un des composants réactifs a) ou b) ou a) et b) d'abord dans la réaction de la première étape est de fonctionnalité supérieure à 2, c'est-à-dire quand une structure ramifiée est visée. Dans ce cas, il est possible de contrôler la structure sans aucune réticulation possible en ajustant les proportions des réactifs de sorte que la fonctionnalité moyenne en nombre (par mole de composant réactant) sur l'ensemble des composants réactifs (a) + b)) ne dépasse pas 2 ou si elle dépasse 2 de limiter le taux de conversion bien avant le point de gel (gélification) prédictible, soit par l'expérimentation, soit par le calcul selon la relation de Macosko-Miller et/ou par addition progressive du composant le moins fonctionnalisé sur le composant le plus fonctionnalisé sous agitation efficace (maintenu en excès de fonctions réactives par l'addition progressive du deuxième composant réactif). La relation de Macosko-Miller citée ci-haut est telle que définie selon Macromolecules, vol. 9, pages 199-211 (1976) et est considérée comme bien connue par l'homme du métier. Pour plus de clarté, nous rappelons ci-dessous cette relation qui relie le rapport critique des fonctions réactives pour deux composants réactifs A et B (respectivement a) et b) dans notre cas) au point critique de gel $r_c$ = (fonctions réactives de A) / (fonctions réactives de B), avec la relation suivante liant la fonctionnalité moyenne $f_A$ de A, $f_B$ de B et le taux critique de conversion $x_g$ au point de gélification :

$$r_c * x_g^2 = 1 / [(f_B - 1)*(f_A - 1)]$$

L'objectif est d'ajuster le rapport r = (fonctions réactives de A) / (fonctions réactives de B) de sorte qu'il ne dépasse pas une valeur critique pour $x_g$ proche de 1 (conversion totale des fonctions en défaut).

Ensuite, la même relation est à faire respecter pour la réaction de modification dudit composé intermédiaire c) avec ledit composé d), porteur d'un acrylate et de p acrylates et/ou méthacrylates en remplaçant respectivement les fonctionnalités $f_A$ et $f_B$ par celles de c) et de d). Il est évident que dans le cas où un seul groupement acrylate est présent dans le composé d) le risque de gélification n'existe pas par réaction amine-acrylate car les groupements méthacrylates ne sont pas réactifs par addition de Michael. Donc, la relation est à considérer dans le cas où au moins deux acrylates sont présents dans d) et que la fonctionnalité de la polyamine a) est d'au moins 3 en -NH-. Il est à noter ici que dans la fonctionnalité de c) en groupements réactifs, -NH-, est de deux pour chaque groupement résiduel réactif amine primaire et de un pour chaque groupement résiduel réactif amine secondaire. Par conséquent, à la fois les proportions et fonctionnalités de a) et de b) dans la première étape de réaction de formation de m uréthane-amines par molécule dudit composé c) comme les proportions et fonctionnalités dudit composé c) (groupements résiduels réactifs -NH-) et ledit composé d) (acrylate) lors de la deuxième étape de réaction (formation d'aminoacrylate par addition de -NH- sur acrylate de d) respectent dans chacune desdites deux étapes, ladite relation adaptée (appliquée) à chacune desdites étapes.

Concernant sa fonctionnalité en acrylates et/ou méthacrylates, ledit oligomère selon la présente invention peut avoir une fonctionnalité f en groupements acrylates ou méthacrylates, laquelle peut varier de 1 à 30, avec f = 1 si monofonctionnel, de préférence oligomère multifonctionnel de fonctionnalité f allant de 2 à 20, plus particulièrement de 3 à 20.

Plus particulièrement, concernant p qui caractérise le nombre de fonctions acrylates et/ou méthacrylates portées par liaison carbone-azote (imine) formée par addition de Michael, ce paramètre p peut varier de 1 à 10, en particulier p = 10 avec des oligomères multiacrylés de préférence de 1 à 5, en particulier p = 5 avec monomères MFA tels que le dipentaérythritol comme composé d) tel que défini selon l'invention.

En fait, la liaison carbone-azote (imine) formée par ladite addition de Michael en seconde étape correspond à un groupement $-N-CH_2-CH_2-CO_2-R_5(-O_2C-CH=CH-R_6)_p$ avec $R_6$ étant H ou méthyle ou H et méthyle si p différent de 1, lequel groupement est un aminoacrylate porteur de p groupements acrylates et/ou méthacrylates.

Selon un cas particulier plus préféré, ledit oligomère selon l'invention correspond à m = 2 et p = 2 à 10, de préférence p = 3 à 5.

Selon une option particulière dudit oligomère selon l'invention, le nombre de groupements résiduels réactifs amine -NH- dudit produit intermédiaire c) est égal au nombre de moles dudit composé d).

Selon une autre option particulière dudit oligomère selon l'invention, ledit composé d) est en excès molaire par rapport aux groupements résiduels réactifs amines -NH- dudit produit intermédiaire c) et ainsi ledit composé d) résiduel est présent dans ledit oligomère comme diluant réactif.

L'excès molaire de d) signifie qu'il y a plus d'une mole de composé d) par groupement résiduel réactif amine -NH- dudit produit c). Le taux de composé d) résiduel correspond à cet excès molaire (différence en moles de d)) par rapport auxdits groupements réactifs amine -NH-, lequel excès par définition n'a pas pu réagir avec c).

Selon une définition alternative de l'oligomère de l'invention par rapport à sa structure, il s'agit d'un oligomère uréthane de structure linéaire ou ramifiée, porteur d'un ou de plusieurs groupements acrylates et/ou méthacrylates, en particulier porteur d'au moins deux groupements acrylates et/ou méthacrylates et plus particulièrement porteur de plus de deux groupements acrylates et/ou méthacrylates, ledit oligomère étant caractérisé en ce qu'il comprend au moins un oligomère de structure selon formule générale (III) suivante :

$$(Acr)_p- R_5-O_2C-CH_2-CH_2-(R_7)N-A-U-R_3[-U-A-[-N(R_7)-CH_2-CH_2-CO_2- R_5-(Acr)_p]_{n-1}]_{m-1},$$

avec 'Acr': groupement acrylate ou méthacrylate

$R_5$ : résidu d'un composé d) porteur d'un groupement acrylate et de p groupements supplémentaires acrylates et/ou méthacrylates, avec p étant égal à au moins 1, en particulier avec d) et $R_5$ étant définis comme décrits ci-haut selon l'invention.

'A' : résidu de polyamine telle que définie selon a) comme décrite ci-haut selon l'invention, porteuse de n groupements amines secondaires ou primaires, avec n allant de 2 à 5,

$R_3$ : résidu de carbonate b) porteur de m groupements carbonates cycliques à 5 ou 6 atomes, avec m allant de 1 à 2, en option ledit résidu étant hydroxylé et/ou ledit cycle carbonate étant substitué, en particulier avec b) et $R_3$ étant définis comme décrits ci-haut selon l'invention,
'$R_7$' : alkyl en $C_1$-$C_3$ ou cycloalkyle, si amine secondaire ou $R_7$: -$CH_2$-$CH_2$-$CO_2$- $R_5$-$(Acr)_p$, si amine primaire,
'Acr' : acrylate ou méthacrylate ou mélange acrylate + méthacrylate
U : liaison uréthane formée par réaction carbonate cyclique avec amine secondaire (-NH-) ou primaire (-NH2), avec un OH en position beta ou gamma en fonction du nombre d'atomes du cycle carbonate à respectivement 5 ou 6 atomes.

Le second objet de l'invention concerne un procédé de préparation dudit oligomère tel que défini ci-haut selon la présente invention, lequel procédé comprend les étapes suivantes :

i) réaction entre ladite polyamine a) et ledit composé carbonate b) porteur de m carbonates cycliques, avec a) et b) étant tels que définis ci-avant selon l'invention, ladite polyamine étant en excès stœchiométrique par rapport à b) et étant ajoutée progressivement sur ledit carbonate b) présent au départ dans le réacteur et ladite réaction donnant un produit intermédiaire c) porteur de m groupements uréthanes et de m groupements hydroxyles en alpha ou beta dudit uréthane et porteur de groupements résiduels amine -NH-
ii) réaction de modification dudit composé c), par addition de chacun desdits groupements résiduels réactifs amine dudit produit c) sur un groupement acrylate d'un composé d) porteur en plus dudit groupement acrylate, de p groupements supplémentaires acrylates et/ou méthacrylates, avec chaque groupement résiduel réactif amine -NH- dudit produit c) étant ainsi transformé en liaison carbone-azote porteuse desdits groupements acrylates et/ou méthacrylates et obtention ainsi dudit oligomère uréthane,
avec

- ladite polyamine a) étant porteuse de n groupements amine primaire et/ou secondaire et en option d'au moins un groupement amine tertiaire, avec n allant de 2 à 5, de préférence de 2 à 4 et plus préférentiellement de 2 à 3, encore plus préférentiellement de 2,
- ledit carbonate b) étant porteur de m groupements carbonates avec m allant de 1 à 2, de préférence le cycle desdits groupements carbonates cycliques étant un cycle à 5 ou à 6 atomes,
- ledit produit intermédiaire c) étant porteur de m groupements uréthanes et de m groupements hydroxyles en alpha ou beta dudit uréthane et de m*(n-1) à m*(2n-2) groupements résiduels réactifs amine -NH-,
- ledit composé d) étant porteur de p groupements acrylate et/ou méthacrylate en plus dudit groupement acrylate qui réagit avec un desdits groupements résiduels réactifs amine -NH- avec p étant au moins égal à 1 et de préférence au moins égal à 2 et
- le nombre desdits groupements résiduels amine -NH- réactifs étant inférieur ou égal au nombre de moles dudit composé d),
- chaque liaison carbone-azote formée étant porteuse de p groupements acrylates et/ou méthacrylates et ledit oligomère uréthane ayant une fonctionnalité en acrylates et/ou méthacrylates allant de m*p(n-1) à m*p(2n-2).

Plus particulièrement, la réaction de l'étape i) peut être réalisée à une température allant de 20 à 80°C, de préférence de 45 à 65°C et celle de l'étape ii) de 80 à 95°C, de préférence de 80 à 90°C.
La présente divulgation concerne également ledit produit intermédiaire c) qui est un multiuréthane-amine hydroxylé, tel que défini ci-haut comme produit de la première étape de réaction i) comme décrite dans la définition du produit oligomère uréthane de l'invention ou du procédé de préparation dudit oligomère selon la présente invention.
Plus particulièrement, ledit produit est le produit intermédiaire c) de la réaction i) entre ladite polyamine a) telle que définie ci-avant et ledit carbonate b), tel que défini ci-avant ou le même produit c) tel qu'obtenu à la fin de l'étape i) du procédé de préparation dudit oligomère selon l'invention.
Un autre objet de l'invention concerne une composition réticulable, laquelle composition comprend comme liant au moins un oligomère tel que défini ci-haut ou tel qu'obtenu par un procédé tel que défini ci-avant selon l'invention. Plus particulièrement, ladite composition est réticulable par voie de rayonnement et/ou par voie peroxyde thermique ou à basse température et/ou par voie d'addition type Michael, en particulier dans le cas d'oligomères portant au moins deux fonctions acrylates et/ou par autre voie duale laquelle peut faire intervenir au moins deux des voies précitées et/ou tout ou une partie des m groupements hydroxyles résiduels dudit oligomère par réaction de ces derniers avec un agent réticulant réactif avec lesdits hydroxyles. Lesdits hydroxyles sont secondaires et en position beta ou gamma de la liaison uréthane formée par réaction entre fonction carbonate cyclique et fonction amine beta ou gamma dépendant du nombre (respectivement 5 ou 6) d'atomes du cycle carbonate de b). En particulier, l'hydroxyle secondaire est en position beta (cycle à 5 atomes pour b)). Comme tels agents convenables, on peut citer des dianhydrides ou la mélamine, en particulier les dianhydrides.
Par rapport à son usage potentiel, ladite composition réticulable de l'invention peut être une composition de revêtements, de préférence revêtements parmi peintures, vernis et encres, une composition de moulage, une composition d'agent d'étanchéité ou de scellement chimique, une composition d'adhésifs, une composition pour systèmes de fabrication d'objets en 3D couche par couche, une composition pour systèmes d'impression en 3D, une composition de béton ou une composition de composite.

Un autre objet de l'invention concerne l'utilisation d'un oligomère tel que défini ci-haut selon l'invention ou obtenu par le procédé de l'invention décrit ci-haut, ledit oligomère étant utilisé comme liant réactif dans les compositions de revêtements, dans ce cas incluant peintures, vernis et encres ou composition d'adhésifs, de moulage, d'agent d'étanchéité, de scellement chimique, de systèmes de fabrication d'objets en 3D couche par couche, de systèmes d'impression en 3D, de béton ou de composite.

Finalement, la présente invention couvre également le produit fini, qui résulte de l'utilisation d'au moins un oligomère tel que défini selon l'invention ou obtenu par le procédé de l'invention décrit ci-haut, de préférence produit choisi parmi les revêtements, en particulier revêtements pour peintures, vernis et encres ou produit parmi les joints adhésifs, les pièces moulées, les objets en 3D obtenus couche par couche, les impressions en 3D, les joints d'étanchéité, le scellement chimique, le béton fini ou les articles en composite.

Les exemples qui suivent sont donnés à titre d'illustration de l'invention et de ses performances et ne limitent nullement sa couverture.

## Partie expérimentale

### 1) Préparation d'oliaomères selon l'invention

#### Exemple 1

Dans un réacteur de 1L, on introduit 161,55 g de carbonate de propylène (Huntstman Jeffsol, Mw de 102 g/mol), 0,81 g de trisnonylphénylphosphite et 1 g de 2,6-di-tert-butyl-4-méthylphénol (BHT). Sous agitation et bullage d'azote, 146,80 g de N-méthyl-1,3-propane diamine (Aldrich, Mw de 88,15 g/mol) sont ajoutés sur une heure à débit constant. Une exothermie d'environ 30°C est observée. A la fin de l'ajout, la température du mélange est portée à 60°C. Après une heure à 60°C, sous bullage d'air, 689,85 g de tripropylène glycol diacrylate (TPGDA, Sartomer SR306, Mw de 300 g/mol) sont ajoutés au mélange en quinze minutes à débit constant. A la fin de l'ajout, la température du mélange est portée à 85-90°C.

L'avancement de la réaction est suivi par mesure des indices d'amine total et d'amine tertiaire. La réaction est arrêtée lorsque l'indice d'amine tertiaire = indice d'amine total.

#### Exemple 2

Dans un réacteur de 1L, on introduit 162,93 g de carbonate de propylène (Huntstman Jeffsol, Mw de 102 g/mol), 0,81 g de trisnonylphénylphosphite et 1 g de 2,6-di-tert-butyl-4-méthylphénol (BHT). Sous agitation et bullage d'azote, 147,81 g de N-méthyl-1,3-propane diamine (Aldrich, Mw de 88,15 g/mol) sont ajoutés sur une heure à débit constant. Une exothermie d'environ 30°C est observée. A la fin de l'ajout, la température du mélange est portée à 60°C. Après une heure à 60°C, sous bullage d'air, 687,44 g de triméthylolpropane triacrylate (TMPTA, Sartomer SR351, Mw de 296 g/mol) sont ajoutés au mélange en quinze minutes à débit constant. A la fin de l'ajout, la température du mélange est portée à 85-90°C.

L'avancement de la réaction est suivi par mesure des indices d'amine total et d'amine tertiaire. La réaction est arrêtée lorsque l'indice d'amine tertiaire = indice d'amine total.

#### Exemple 3

Dans un réacteur de 1L, on introduit 336,04 g de Polyéthylène oxide dicarbonate (Specific Polymers, Mw de 632,5 g/mol), 1,68 g de trisnonylphénylphosphite et 2,07 g de 2,6-di-tert-butyl-4-méthylphénol (BHT). Sous agitation et bullage d'azote, 95,38 g de N-méthyl-1,3-propane diamine (Aldrich, Mw de 88,15 g/mol) sont ajoutés sur une heure à débit constant. Une exothermie d'environ 30°C est observée. A la fin de l'ajout, la température du mélange est portée à 60°C. Après une heure à 60°C, sous bullage d'air, 564,84 g de triméthylolpropane triacrylate (TMPTA, Sartomer SR351, Mw de 296 g/mol) sont ajoutés au mélange en quinze minutes à débit constant. A la fin de l'ajout, la température du mélange est portée à 85-90°C.

L'avancement de la réaction est suivi par mesure des indices d'amine total et d'amine tertiaire. La réaction est arrêtée lorsque l'indice d'amine tertiaire = indice d'amine total.

2) Caractéristiques des produits préparés

| Caractéristiques | Unité | Exemple 1 | Exemple 2 | Exemple 3 |
|---|---|---|---|---|
| Viscosité Noury | Pa.s | 2,0 (25°C) | 2,4 (50°C) | 12,3 (50°C) |
| Vitesse de réticulation sous lampe UV | m/min | < 10 | 50 | > 80 |
| Dureté Persoz | Nombre d'oscillations | 60 | 75 | 103 |
| Flexibilité | mm | N/DA | 20 | 8 |
| Résistance à l'acétone | s | 10 | > 300 | > 300 |

Les propriétés applicatives sont mesurées sur un film réticulé sous lampe UV « fusion » 120 W/cm à partir d'un mélange uréthane amino-acrylate selon l'invention / photoinitiateur Darocur® 1173 dans les proportions 96/4 w/w.

Méthodes utilisées

Détermination de la réactivité (vitesse de réticulation) : Le mélange est appliqué en film de 12 μm sur une carte contraste (« Penoparc charts form 1B » Leneta), puis est réticulé à l'aide d'une lampe fusion Hg 120 W/cm. On mesure la vitesse de passage minimum nécessaire (en m/min) pour obtenir un film sec au toucher.

Pour les tests suivants de dureté, flexibilité et de résistance à l'acétone, les films photoréticulés sont laissés en salle climatisée (T = 23°C) pendant 24 heures après réticulation et avant les mesures.

Détermination de la dureté Persoz : Le mélange est appliqué en film de 100 μm sur une plaque de verre et réticulé par une lampe fusion Hg 120 W/cm à une vitesse de 8m/min. On mesure le nombre d'oscillations avant l'amortissement des oscillations (passage de 12° à 4° d'amplitude), d'un pendule au contact de la plaque de verre revêtue d'après la norme ISO 1522.

Détermination de la flexibilité : Le mélange est appliqué en film de 100 μm sur une plaque lisse de 25/100 mm d'épaisseur (D-46 Q-Panel), puis réticulé par une lampe fusion Hg 120 W/cm à une vitesse de 8m/min. On courbe la plaque revêtue sur des mandrins cylindriques d'après la norme ISO 1519. On exprime le résultat par la valeur (en mm) du rayon de courbure le plus faible qu'on peut infliger au revêtement sans qu'il ne fissure, ni se décolle du support.

Détermination de la résistance à l'acétone : Le mélange est appliqué en film de 12 μm sur une plaque de verre, puis réticulé par une lampe fusion Hg 120 W/cm à une vitesse de 8m/min. On frotte le revêtement avec un chiffon imbibé d'acétone. Le résultat est le temps (exprimé en seconde) au-delà duquel le film se décolle et/ou se désagrège.

**Revendications**

**1.** Oligomère uréthane monofonctionnel ou multifonctionnel en acrylates et/ou méthacrylates, de préférence acrylates, lequel est susceptible d'être obtenu à partir de la réaction d'une polyamine spécifique a) avec un composé carbonate cyclique b) porteur de m groupements carbonates cycliques, ladite polyamine étant en excès stœchiométrique par rapport à b) et ladite réaction donnant un produit intermédiaire c) porteur de m groupements uréthanes formés porteurs de groupements résiduels réactifs amine -NH- et ensuite par une réaction d'addition de chacun desdits groupements résiduels réactifs amine dudit produit c) sur un groupement acrylate d'un composé d) porteur en plus dudit groupement acrylate de p groupements supplémentaires acrylates et/ou méthacrylates, avec chaque groupement résiduel réactif amine -NH- dudit produit c) étant ainsi transformé en liaison carbone-azote porteuse desdits groupements acrylates et/ou méthacrylates et obtention ainsi dudit oligomère uréthane, avec :

- ladite polyamine a) étant porteuse de n groupements amine primaire et/ou secondaire et en option d'au moins un groupement amine tertiaire, avec n allant de 2 à 5, de préférence de 2 à 4 et plus préférentiellement de 2 à 3, encore plus préférentiellement de 2,
- ledit carbonate b) étant porteur de m groupements carbonates cycliques avec m allant de 1 à 2, de préférence le cycle desdits groupements carbonates cycliques étant un cycle à 5 ou à 6 atomes,
- ledit produit intermédiaire c) étant porteur de m groupements uréthanes et de m groupements OH en alpha ou beta dudit groupement uréthane et c) étant porteur de m*(n-1) à m*(2n-2) groupements résiduels réactifs amine -NH-,
- ledit composé d) étant porteur de p groupements acrylate et/ou méthacrylate en plus dudit groupement acrylate qui réagit avec un desdits groupements résiduels réactifs amine -NH- avec p étant au moins égal à 1 et de préférence au moins égal à 2 et
- le nombre desdits groupements résiduels réactifs amine -NH- dudit produit c) étant inférieur ou égal au nombre de moles dudit composé d),
- chaque liaison carbone-azote formée étant porteuse de p groupements acrylates et/ou méthacrylates et ledit oligomère uréthane étant porteur de m groupements uréthanes et de m groupement hydroxyles en alpha ou beta dudit uréthane et ayant une fonctionnalité en acrylates et/ou méthacrylates allant de m*p(n-1) à m*p(2n-2)

ladite polyamine a) étant choisie parmi les polyamines de formules a1) ou a2) suivantes :

a1) $R_1$-NH-$R_2$-(NH-R)(NH-R')$_{n1}$, avec $n_1$ étant égal à 3, 2, 1 ou 0
a2) $R_1$-NH-(R'$_2$NH)$_{n2}$-R"$_2$-NH-R' avec $n_2$ étant égal à 3, 2 ou 1

avec $R_1$ et R et R' étant choisis indépendamment parmi : H, alkyle de préférence $C_1$-$C_3$, en option ledit alkyle étant alcoxylé ou un cyclolalkyle, avec $R_1$ pouvant être identique ou différent de R et R' et avec R et R' pouvant être identiques ou différents et en particulier avec R et/ou R' étant différents de H,
$R_2$ étant alkylène ou cycloalkylène (comprenant un cycle aliphatique en $C_6$) ou aralkylène de valence n allant de 2 à 5, avec $R_2$ pouvant porter ou comprendre au moins un groupement amine tertiaire
et

R'2 et R"$_2$ pouvant être identiques ou différents et choisis parmi alkylènes, de préférence en C2 à $C_8$, plus préférentiellement en $C_2$ à $C_6$ et si R"$_2$ est différent de R'$_2$ dans ce cas R"$_2$ pouvant porter ou comprendre au moins un groupement amine tertiaire.

**2.** Oligomère selon la revendication 1, **caractérisé en ce que** lesdits n groupements amine primaire et/ou secondaire de ladite polyamine a) peuvent être portés par un radical A de valence n, lequel radical A peut être ou comprendre une structure aliphatique, cycloaliphatique ou aromatique avec dans ce dernier cas lesdits n groupements amines étant portés par au moins un groupement alkylène attaché au noyau aromatique et les cycles desdits m groupements carbonates cycliques dudit carbonate b) sont à 5 ou 6 atomes et portés par un radical $R_3$ de valence m, avec $R_3$ étant un radical hydrocarboné de structure aliphatique, cycloaliphatique ou aromatique et en option hydroxylé ou $R_3$ peut être un simple hydrogène pour m = 1, en option le cycle carbonate peut être substitué par un substituant $R_4$ hydrocarboné éventuellement hydroxylé, $R_4$ pouvant être de structure aliphatique, cycloaliphatique ou aromatique identique ou différente de celle de $R_3$ et éventuellement pouvant former un cycle attaché au cycle carbonate par deux atomes de carbone communs.

**3.** Oligomère selon la revendication 1 ou 2, **caractérisé en ce que** ladite polyamine a) est choisie parmi :

- une alkylène polyamine en $C_2$ à $C_{54}$, ledit alkylène étant en $C_2$ à $C_{54}$ linéaire ou ramifié et porteur desdits n groupements amine primaire et/ou secondaire,
- une polyamine comprenant au moins deux unité répétitives parmi : alkylène imine en $C_2$ à $C_4$, en particulier éthylène imine ou propylène imine, de préférence éthylène imine, ladite polyamine a) étant dans ce cas une oligoalkylène imine polyamine, en particulier une polyéthylène imine polyamine y compris de structure dendritique,
- une polyamine comprenant une ou plusieurs unités parmi : éther, ester-amide, amide, avec dans le cas de présence de plusieurs unités, ladite polyamine a) étant choisi parmi : oligoéthers polyamines, oligoester amide-polyamines ou multiester amide-polyamines.

**4.** Oligomère selon l'une des revendications 1 à 3, **caractérisé en ce que** ladite polyamine a) est choisie parmi les polyamines de formule a1) avec $n_1$ étant égal à 2, 1 ou 0 ou parmi les polyamines de formule a2) avec $R_1$ et R et R' étant choisis indépendamment parmi alkyle en $C_1$-$C_3$, en option ledit alkyle étant alcoxylé ou un cyclolalkyle, avec $R_1$ pouvant être identique ou différent de R et R' et avec R et R' pouvant être identiques ou différents et avec R et/ou R' étant différents de H.

**5.** Oligomère selon l'une des revendications 1 à 4, **caractérisé en ce que** ledit carbonate b) est un ester complet ou partiel d'un polyacide, monomérique ou oligomérique tel qu'un oligoester polyacide, avec un carbonate hydroxylé, de préférence le carbonate de glycérol ou b) est un éther complet ou partiel d'un polyol monomérique ou oligomérique tel que oligoester ou oligoéther polyol avec un carbonate hydroxylé, de préférence le carbonate de glycérol ou b) est un éther complet ou partiel d'un dérivé phénolique, y compris bisphénol A ou dihydroxyphénylène avec un carbonate hydroxylé, de préférence le carbonate de glycérol ou b) est un éther d'alcool allylique ou vinylique avec un carbonate hydroxylé, de préférence le carbonate de glycérol ou un méthacrylate d'un carbonate hydroxylé, de préférence du carbonate de glycérol ou b) est un carbonate obtenu par addition de $CO_2$ sur un composé précurseur monomérique ou oligomérique époxydé ou b) est le produit de la réaction du diméthylcarbonate avec un polyol aliphatique ou cycloaliphatique de fonctionnalité d'au moins 2, de préférence allant de 2 à 6.

**6.** Oligomère selon l'une des revendications 1 à 5, **caractérisé en ce que** ledit composé d) est représenté globalement par $R_5$-($O_2CCH=CH_2$)(-$O_2CC(R_6)=CH_2$)$_p$ avec $R_5$ étant un résidu hydrocarboné de valence p+1 et $R_6$ est H ou méthyle pour une partie ou la totalité de p groupements et de préférence $R_5$ étant un résidu de polyol choisi parmi : alkylène polyol en option alkoxylé et/ou substitué, cycloalkylène polyol en option substitué ou aralkylène polyol en option alcoxylé et/ou substitué sur le cycle aromatique ou polyéther polyol ou polyester polyol ou $R_5$ est un résidu d'un composé époxy acrylate multifonctionnel porteur de un groupement acrylate et de p groupements supplémentaires acrylates et/ou méthacrylates.

**7.** Oligomère selon l'une des revendications 1 à 6, **caractérisé en ce que** ladite polyamine a) est une diamine.

**8.** Oligomère selon l'une des revendications 4 à 7, **caractérisé en ce que** le cycle carbonate dudit carbonate b) est à 5 atomes, que ladite polyamine a) est une diamine secondaire-primaire selon formule a1) telle que définie selon la revendication 1, avec $n_1 = 0$ et R = H et que ledit oligomère comprend au moins un oligomère de formule générale (I) suivante :

$$[(CH_2=CH(R_6)-CO_2)_p-R_5-O_2C-CH_2-CH_2-N(R_1)-R_2-NH-CO_2-C(R_4)-CH(OH)-]_mR_3 \qquad (I)$$

avec

$R_1$ étant tel que défini selon la revendication 1
R2 étant tel que défini selon la revendication 1
R3 étant tel que défini selon la revendication 2
$R_4$ étant tel que défini selon la revendication 2
$R_5$ étant tel que défini selon la revendication 7
$R_6$ étant H ou méthyle ou H et méthyle si p est différent de 1.

**9.** Oligomère selon l'une des revendications 1 à 7, **caractérisé en ce que** ledit cycle carbonate dudit carbonate b) est à 6 atomes, que ladite polyamine a) est une diamine secondaire-primaire selon formule a1) comme définie dans la revendication 1, avec $n_1 = 0$ et R = H et que ledit oligomère comprend au moins un oligomère de formule générale (II) suivante :

$$[(CH_2=CH(R_6)-CO_2)_p-R_5-O_2C-CH_2-CH_2-N(R_1)-R_2-NH-CO_2-C(R_4)-CH_2CH(OH)-]_mR_3 \qquad (II)$$

avec $R_1$, $R_2$, $R_3$, $R_4$, $R_5$ et $R_6$ étant tels que définis selon la revendication 8.

**10.** Oligomère selon l'une des revendications 1 à 7, **caractérisé en ce que** ladite polyamine a) est une diamine sélectionnée parmi les diamines secondaire-primaire, primaire-primaire ou secondaire-secondaire, de préférence secondaire-primaire ou secondaire-secondaire.

**11.** Oligomère selon la revendication 10, **caractérisé en ce que** ladite diamine est sélectionnée parmi les diamines secondaire-primaire, de préférence parmi : N-méthyl 1,3-propane diamine, N-méthyl éthane diamine, N-méthyl 1,4-butane diamine, N-méthyl 1,5-pentane diamine.

**12.** Oligomère selon la revendication 10, **caractérisé en ce que** ladite diamine est sélectionnée parmi les diamines secondaire-secondaire, de préférence parmi : N-méthyl N'méthyl 1,3-propane diamine, N-méthyl N'méthyl éthane diamine, N-méthyl, N'méthyl 1,4-butane diamine, N-méthyl, N'méthyl 1,5- pentane diamine.

**13.** Oligomère selon l'une des revendications 4 à 7, **caractérisé en ce que** ladite polyamine a) est une amine selon formule a2) telle que définie selon la revendication 1, avec $n_2 = 1$ et $R_1$ et R' étant H et $R'_2$ et $R''_2$ étant des alkylènes identiques ou différents et en $C_2$ à $C_6$, en particulier en $C_2$ à $C_4$, plus particulièrement ladite amine a2) étant sélectionnée parmi 3(2-aminoéthyl)aminopropylamine ou bis(3-aminopropyl)amine.

**14.** Oligomère selon l'une des revendications 1 à 7, **caractérisé en ce que** ladite polyamine a) est une amine selon formule a2) telle que définie selon la revendication 1,
avec $n_2$ = 2 ou 3 et $R_1$ et R' étant H et $R'_2$ et R"2 étant des alkylènes identiques en $C_2$ à $C_4$, en particulier en $C_2$ à $C_3$, plus particulièrement ladite amine a2) étant sélectionnée parmi triéthylènetetramine ou tetraéthylènepentamine.

**15.** Oligomère selon l'une des revendications 1 à 14, **caractérisé en ce que** ledit composé d) est un monomère acrylique multifonctionnel ayant une fonctionnalité d'au moins 2, en particulier supérieure à 2 et allant jusqu'à p+1 égal à 6 et sélectionné parmi :

d1) les esters acrylates de polyols alcoxylés ou non alcoxylés, de préférence polyols choisis dans le groupe : trimethylol propane, ditrimethylol propane, pentaérythritol, dipentaérythritol, diéthylène glycol, dipropylène glycol, tripropylène glycol, glycérol, propylène glycol, butane diol, hexane diol ou
d2) les époxy acrylates,
d3) les aminoacrylates.

**16.** Oligomère selon l'une des revendications 1 à 14, **caractérisé en ce que** ledit composé d) est un oligomère acrylique multifonctionnel ayant une fonctionnalité d'au moins 2, en particulier supérieure à 2 et allant jusqu'à p+1 égal à 12 et sélectionné parmi :

d4) oligomère acrylique acrylé qui peut être un copolymère de glycidyl méthacrylate acrylé ou oligomère styrénique acrylé qui peut être un copolymère de styrène avec l'anhydride maléique ou l'acide acrylique qui est acrylé par l'hydroxy éthyl acrylate,
d5) oligomère hydroxylé acrylé, en particulier parmi polydiènes hydroxylés acrylés, de préférence le polybutadiène hydroxylé acrylé et plus préférentiellement le polybutadiène hydroxylé hydrogéné acrylé
d6) huile époxydé acrylée
d7) oligodiène époxydé acrylé
d8) oligoéthers acrylates
d9) oligoesters acrylates
d10) oligoaminoacrylates.

**17.** Oligomère selon l'une des revendications 1 à 16, **caractérisé en ce que** la fonctionnalité f en groupements acrylates et/ou méthacrylates varie de 1 à 30, avec f = 1 si monofonctionnel, de préférence oligomère multifonctionnel de fonctionnalité f allant de 2 à 20, plus particulièrement de 3 à 20.

**18.** Oligomère selon l'une des revendications 1 à 17, **caractérisé en ce que** p varie de 1 à 11, de préférence de 2 à 11, plus préférentiellement de 2 à 5, encore plus préférentiellement étant supérieur à 2 et allant jusqu'à 5.

**19.** Oligomère selon l'une des revendications 1 à 18, **caractérisé en ce que** m = 2 et que p varie de 2 à 11 et de préférence p varie de 3 à 5.

**20.** Oligomère selon l'une des revendications 1 à 19, **caractérisé en ce que** ledit composé d) est en excès molaire par rapport aux groupements amines -NH- dudit produit intermédiaire c) et avec ledit composé d) résiduel étant présent dans ledit oligomère comme diluant réactif.

**21.** Procédé de préparation d'un oligomère tel que défini selon l'une des revendications 1 à 20, **caractérisé en ce qu'**il comprend les étapes suivantes :

i) réaction entre ladite polyamine a) telle que définie selon l'une des revendications 1 à 4, 7 à 14 et 19 et ledit composé carbonate b) porteur de m carbonates cycliques, tel que défini selon l'une des revendications 1, 2, 5, 8, 9 et 19, ladite polyamine étant en excès stœchiométrique par rapport à b) et étant ajoutée progressivement sur ledit carbonate b) présent au départ dans le réacteur et ladite réaction donnant un produit intermédiaire c) porteur de m groupements uréthanes et de m groupements hydroxyles en alpha ou beta dudit uréthane et porteur de groupements résiduels réactifs amine-NH-
ii) réaction de modification dudit composé c), par addition de chacun desdits groupements résiduels réactifs amine dudit produit c) sur un groupement acrylate d'un composé d) porteur en plus dudit groupement acrylate, de p groupements supplémentaires acrylate et/ou méthacrylate tel que défini selon l'une des revendications 1, 6, 15 et 16, avec chaque groupement résiduel réactif amine -NH- dudit produit c) étant ainsi transformé en liaison carbone-azote porteuse desdits groupements acrylates et/ou méthacrylates et obtention ainsi dudit oligomère uréthane,
avec

- ladite polyamine a) étant porteuse de n groupements amine primaire et/ou secondaire et en option d'au moins un groupement amine tertiaire, avec n allant de 2 à 5, de préférence de 2 à 4 et plus préférentiellement de 2 à 3, encore plus préférentiellement de 2,
- ledit carbonate b) étant porteur de m groupements carbonates avec m allant de 1 à 2, de préférence le cycle desdits groupements carbonates cycliques étant un cycle à 5 ou à 6 atomes,
- ledit produit intermédiaire c) étant porteur de m groupements uréthanes et de m groupements hydroxyles en alpha ou beta dudit uréthane et de m*(n-1) à m*(2n-2) groupements résiduels amine -NH-,
- ledit composé d) étant porteur de p groupements acrylate et/ou méthacrylate en plus dudit groupement acrylate qui réagit avec un desdits groupements résiduels réactifs amine -NH- avec p étant au moins égal à 1 et de préférence au moins égal à 2 et
- le nombre desdits groupements résiduels réactifs amine -NH- étant inférieur ou égal au nombre de moles dudit composé d),
- chaque liaison carbone-azote formée étant porteuse de p groupements acrylates et/ou méthacrylates et ledit oligomère uréthane ayant une fonctionnalité en acrylates et/ou méthacrylates allant de m*p(n-1) à m*p(2n-2).

**22.** Composition réticulable, **caractérisée en ce qu'**elle comprend comme liant au moins un oligomère tel que défini selon l'une des revendications 1 à 20 ou tel qu'obtenu par un procédé tel que défini selon la revendication 21.

**23.** Composition réticulable selon la revendication 22, **caractérisée en ce qu'**elle est réticulable par voie de rayonnement et/ou par voie peroxyde thermique ou à basse température et/ou par voie d'addition type Michael dans le cas d'oligomères portant au moins deux fonctions acrylates et/ou par autre voie duale faisant intervenir au moins deux des voies citées et/ou faisant intervenir la totalité ou une partie des m groupements hydroxyles résiduels dudit oligomère, par réaction de ces derniers avec un agent réticulant réactif avec lesdits hydroxyles.

**24.** Composition réticulable selon la revendication 22 ou 23, **caractérisée en ce qu'**il s'agit d'une composition de revêtements, de préférence parmi revêtements pour peintures, vernis et encres, d'une composition de moulage, d'une composition d'agent d'étanchéité ou de scellement chimique, d'une composition d'adhésifs, d'une composition pour systèmes de fabrication d'objets en 3D couche par couche, d'une composition pour systèmes d'impression en 3D, d'une composition de béton ou d'une composition de composite.

**25.** Utilisation d'un oligomère tel que défini selon l'une des revendications 1 à 20 ou obtenu par le procédé tel que défini selon la revendication 21, comme liant réactif dans des compositions de revêtements en particulier peintures, vernis et encres, d'adhésifs, de moulage, d'agent d'étanchéité, de scellement chimique, pour systèmes de fabrication d'objets en 3D couche par couche, pour systèmes d'impression en 3D, de béton ou de composite.

**26.** Produit fini, **caractérisé en ce qu'**il résulte de l'utilisation d'au moins un oligomère tel que défini selon l'une des revendications 1 à 20 ou obtenu par le procédé tel que défini selon la revendication 21, de préférence produit choisi parmi les revêtements, en particulier revêtements pour peintures, vernis et encres, produit parmi les joints adhésifs, les pièces moulées, les objets en 3D obtenus couche par couche, les impressions en 3D, les joints d'étanchéité, le scellement chimique, le béton fini ou les articles en composite.

**Patentansprüche**

**1.** Urethanoligomer mit einer oder mehreren funktionellen Gruppen des Typs Acrylat und/oder Methacrylat, vorzugsweise Acrylat, wobei es erhalten werden kann, indem ein spezifisches Polyamin a) mit einer zyklischen Carbonatverbindung b) umgesetzt wird, welche mit m zyklischen Carbonatgruppen versehen ist, wobei das Polyamin im stöchiometrischen Überschuss gegenüber b) vorliegt und die Reaktion ein Zwischenprodukt c) ergibt, das mit m Urethangruppen versehen ist, welche nach ihrer Bildung über verbleibende reaktionsfähige Amingruppen -NH-verfügen, wobei anschließend eine Additionsreaktion jeder der verbleibenden reaktionsfähigen Amingruppen des Produkts c) an eine Acrylatgruppe einer Verbindung d) erfolgt, welche über diese Acrylatgruppe hinaus mit p zusätzlichen Acrylat- oder Methacrylatgruppen versehen ist, wobei auf diese Weise jede der verbleibenden reaktionsfähigen Amingruppen -NH- des Produkts c) in eine Kohlenstoff-Stickstoff-Bindung umgewandelt wird, welche mit den Acrylat- und/oder Methacrylatgruppen versehen ist, und auf diese Weise das Urethanoligomer erhalten wird,

wobei:

- das Polyamin a) mit n primären und/oder sekundären Amingruppen sowie möglicherweise mit mindestens einer tertiären Aminogruppe versehen ist, wobei n im Bereich von 2 bis 5, vorzugsweise von 2 bis 4 und stärker bevorzugt von 2 bis 3 liegt, wobei es mit noch größerem Vorzug gleich 2 ist,
- das Carbonat b) mit m zyklischen Carbonatgruppen versehen ist, wobei m im Bereich von 1 bis 2 liegt, wobei es sich bei dem Ring der zyklischen Carbonatgruppen vorzugsweise um einen Ring mit 5 oder mit 6 Atomen handelt,
- das Zwischenprodukt c) mit m Urethangruppen und mit m OH-Gruppen in alpha- oder beta-Stellung zu der Urethangruppe versehen ist und c) mit m*(n-1) bis m*(2n-2) verbleibenden reaktionsfähigen Amingruppen -NH- versehen ist,
- die Verbindung d) über die Acrylatgruppe, welche mit den verbleibenden reaktionsfähigen Amingruppen -NH- reagiert, hinaus mit p Acrylat- und/oder Methacrylatgruppen versehen ist, wobei p mindestens gleich 1 ist und vorzugsweise mindestens gleich 2 ist, und
- die Anzahl der verbleibenden reaktionsfähigen Amingruppen -NH- des Produkts c) höchstens gleich der Molanzahl der Verbindung d) ist,
- jede der Kohlenstoff-Stickstoff-Bindungen nach ihrer Bildung mit p Acrylat- und/oder Methacrylatgruppen versehen ist und das Urethanoligomer mit m Urethangruppen und mit m Hydroxylgruppen in alpha- oder beta-Stellung zu dem Urethan versehen ist und sein Funktionalisierungsgrad bezüglich Acrylaten und/oder Methacrylaten im Bereich von m*p(n-1) bis m*p(2n-2) liegt,

wobei das Polyamin a) aus den Polyaminen mit den folgenden Formeln a1) oder a2) ausgewählt ist:

a1) $R_1$-NH-$R_2$-(NH-R) (NH—R')$_{n1}$, wobei $n_1$ gleich 3, 2, 1 oder 0 ist,
a2) $R_1$-NH-(R'$_2$NH)$_{n2}$-R"$_2$-NH-R', wobei $n_2$ gleich 3, 2 oder 1 ist,

wobei $R_1$ und R und R' unabhängig voneinander aus Folgendem ausgewählt sind: H, Alkyl, vorzugsweise des Typs $C_1$-$C_3$, wobei das Alkyl möglicherweise alkoxyliert ist oder es sich um ein Cycloalkyl handelt, wobei $R_1$ mit R und R' übereinstimmen oder sich davon unterscheiden kann und wobei R und R' vollkommen gleichartig oder verschiedenartig sein können und sich R und/oder R' insbesondere von H unterscheiden, wobei es sich bei $R_2$ um Alkylen oder Cycloalkylen handelt (welches einen aliphatischen $C_6$-Ring umfasst), oder um Aralkylen mit einer Wertigkeit von n, welche im Bereich von 2 bis 5 liegt, wobei $R_2$ mit mindestens einer tertiären Amingruppe versehen sein oder eine solche umfassen kann,
und
wobei R'$_2$ und R"$_2$ vollkommen gleichartig oder verschiedenartig sein können und aus den Alkylenen, vorzugsweise des Typs $C_2$ bis $C_8$, stärker bevorzugt des Typs $C_2$ bis $C_6$, ausgewählt sind, wobei R"$_2$, falls sich R"$_2$ und R'$_2$ voneinander unterscheiden, mit mindestens einer tertiären Amingruppe versehen sein oder eine solche umfassen kann.

**2.** Oligomer nach Anspruch 1, **dadurch gekennzeichnet, dass** sich die n primären und/oder sekundären Amingruppen des Polyamins a) an einem Rest A mit der Wertigkeit n befinden können, wobei es sich bei dem Rest A um eine aliphatische, cycloaliphatische oder aromatische Struktur handeln kann oder er eine solche umfassen kann, wobei sich die n Amingruppen im letztgenannten Fall an mindestens einer Alkylengruppe befinden, welche an den aromatischen Kern gebunden ist und wobei die Ringe der m zyklischen Carbonatgruppen des Carbonats b) aus 5 oder 6 Atomen bestehen und sich an einem Rest $R_3$ mit der Wertigkeit m befinden, wobei $R_3$ ein Kohlenwasserstoffrest mit aliphatischer, cycloaliphatischer oder aromatischer Struktur ist, welcher möglicherweise hydroxyliert ist, oder es sich bei $R_3$ um ein einfaches H handeln kann, wenn m = 1 ist, wobei der Carbonatring möglicherweise mit einem kohlenwasserstoffartigen Substituenten $R_4$ versehen sein kann, welcher möglicherweise hydroxyliert ist, wobei $R_4$ eine aliphatische, cycloaliphatische oder aromatische Struktur aufweisen kann, die mit derjenigen von $R_3$ übereinstimmt oder sich davon unterscheidet, und möglicherweise einen Ring bilden kann, der über zwei gemeinsame Kohlenstoffatome an den Carbonatring gebunden ist.

**3.** Oligomer nach Anspruch 1 oder 2, **dadurch gekennzeichnet, dass** das Polyamin a) aus den folgenden ausgewählt ist:

- einem Alkylenpolyamin des Typs $C_2$ bis $C_{54}$, wobei das Alkylen des Typs $C_2$ bis $C_{54}$ geradkettig oder verzweigt ist und mit den n primären und/oder sekundären Amingruppen versehen ist,
- einem Polyamin, das mindestens zwei Wiederholeinheiten der folgenden umfasst: Alkylenimin des Typs $C_2$

bis $C_4$, insbesondere Ethylenimin oder Propylenimin, vorzugsweise Ethylenimin, wobei es sich bei dem Polyamin a) in diesem Fall um ein Oligoalkylenimin-Polyamin handelt, insbesondere um ein Polyethylenimin-Polyamin, insbesondere ein Polyethylenimin-Polyamin, wobei letztes auch von dendritischer Struktur sein kann,
- einem Polyamin, das eine oder mehrere der folgenden Einheiten umfasst: Ether, Esteramid, Amid, wobei das Polyamin a) im Falle des Vorliegens mehrerer Einheiten aus den folgenden ausgewählt ist: Oligoether-Polyaminen, Oligoesteramid-Polyaminen oder Multiesteramid-Polyaminen.

4. Oligomer nach einem der Ansprüche 1 bis 3, **dadurch gekennzeichnet, dass** das Polyamin a) aus den Polyaminen mit der Formel a1), wobei $n_1$ gleich 2, 1 oder 0 ist, oder aus den Polyaminen mit der Formel a2) ausgewählt ist, wobei $R_1$ und R und R' unabhängig voneinander aus $C_1$-$C_3$-Alkyl ausgewählt sind, wobei das Alkyl möglicherweise alkoxyliert ist oder es sich um ein Cycloalkyl handelt, wobei $R_1$ mit R und R' übereinstimmen oder sich davon unterscheiden kann und wobei R und R' vollkommen gleichartig oder verschiedenartig sein können und sich R und/oder R' von H unterscheiden.

5. Oligomer nach einem der Ansprüche 1 bis 4, **dadurch gekennzeichnet, dass** es sich bei dem Carbonat b) um eine vollständig oder teilweise mit einem hydroxylierten Carbonat, vorzugsweise mit Glycerincarbonat, veresterte Polysäure handelt, die als Monomer oder Oligomer vorliegt, wie etwa um eine Oligoesterpolysäure, oder es sich bei b) um ein vollständig oder teilweise mit einem hydroxylierten Carbonat, vorzugsweise mit Glycerincarbonat, verethertes Polyol handelt, das als Monomer oder Oligomer vorliegt, wie etwa um ein Oligoester- oder Oligoetherpolyol, oder es ich bei b) um ein vollständig oder teilweise mit einem hydroxylierten Carbonat, vorzugsweise mit Glycerincarbonat, verethertes Phenolderivat handelt, wobei dazu Bisphenol A oder Dihydroxyphenylen gehören, oder es sich bei b) um einen Ether von Allyl- oder Vinylalkohol mit einem hydroxylierten Carbonat, vorzugsweise mit Glycerincarbonat, oder um ein Methacrylat eines hydroxylierten Carbonats, vorzugsweise von Glycerincarbonat, handelt oder es sich bei b) um ein Carbonat handelt, welches durch Anlagerung von $CO_2$ an eine epoxidartige Vorstufe erhalten wird, welche als Monomer oder Oligomer vorliegt, oder es sich bei b) um das Produkt der Reaktion von Dimethylcarbonat mit einem aliphatischen oder cycloaliphatischen Polyol handelt, wobei der Funktionalisierungsgrad des letzteren mindestens 2 beträgt und vorzugsweise im Bereich von 2 bis 6 liegt.

6. Oligomer nach einem der Ansprüche 1 bis 5, **dadurch gekennzeichnet, dass** die Verbindung d) in ihrer Gesamtheit durch $R_5$-($O_2CCH=CH_2$) (-$O_2CC$ ($R_6$) =$CH_2$)$_p$ dargestellt ist, wobei $R_5$ ein Kohlenwasserstoffrest mit einer Wertigkeit von p+1 ist und $R_6$ bei einem Teil oder der Gesamtheit der p Gruppen für H oder Methyl steht und wobei es sich bei $R_5$ vorzugsweise um einen Polyolrest handelt, der aus den folgenden ausgewählt ist: Alkylenpolyol, welches möglicherweise alkoxyliert und/oder substituiert ist, Cycloalkylenpolyol, welches möglicherweise substituiert ist, oder Aralkylenpolyol, welches möglicherweise am aromatischen Ring alkoxyliert und/oder substituiert ist, oder Polyetherpolyol oder Polyesterpolyol, oder es handelt sich bei $R_5$ um einen Rest einer multifunktionellen Epoxyacrylatverbindung, welche mit einer Acrylatgruppe und mit p zusätzlichen Acrylat- und/oder Methacrylatgruppen versehen ist.

7. Oligomer nach einem der Ansprüche 1 bis 6, **dadurch gekennzeichnet, dass** es sich bei dem Polyamin a) um ein Diamin handelt.

8. Oligomer nach einem der Ansprüche 4 bis 7, **dadurch gekennzeichnet, dass** der Carbonatring des Carbonats b) aus 5 Atomen besteht, dass es sich bei dem Polyamin a) um ein sekundär-primäres Diamin nach der Formel a1) gemäß der Begriffsbestimmung in Anspruch 1 handelt, wobei $n_1$ = 0 ist und R = H ist, und dass das Oligomer mindestens ein Oligomer mit der folgenden allgemeinen Formel (I) umfasst:

$$[(CH_2=CH(R_6)-CO_2)_p-R_5-O_2C-CH_2-CH_2-N(R_1)-R_2-NH-CO_2-C(R_4)-CH(OH)-]_mR_3 \qquad (I)$$

wobei

$R_1$ der Begriffsbestimmung in Anspruch 1 entspricht
$R_2$ der Begriffsbestimmung in Anspruch 1 entspricht
$R_3$ der Begriffsbestimmung in Anspruch 2 entspricht
$R_4$ der Begriffsbestimmung in Anspruch 2 entspricht
$R_5$ der Begriffsbestimmung in Anspruch 7 entspricht
$R_6$ für H oder Methyl steht oder für H und Methyl, wenn p ungleich 1 ist.

**9.** Oligomer nach einem der Ansprüche 1 bis 7, **dadurch gekennzeichnet, dass** der Carbonatring des Carbonats b) aus 6 Atomen besteht, dass es sich bei dem Polyamin a) um ein sekundär-primäres Diamin nach der Formel a1) gemäß der Begriffsbestimmung in Anspruch 1 handelt, wobei $n_1 = 0$ ist und R = H ist, und dass das Oligomer mindestens ein Oligomer mit der folgenden allgemeinen Formel (II) umfasst:

$$[(CH_2=CH(R_6)\text{-}CO_2)_p\text{-}R_5\text{-}O_2C\text{-}CH_2\text{-}CH_2\text{-}N(R_1)\text{-}R_2\text{-}NH\text{-}CO_2\text{-}C(R_4)\text{-}CH_2CH(OH)\text{-}]_mR_3 \quad (II)$$

wobei $R_1$, $R_2$, $R_3$, $R_4$, $R_5$ und $R_6$ den Begriffsbestimmungen in Anspruch 8 entsprechen.

**10.** Oligomer nach einem der Ansprüche 1 bis 7, **dadurch gekennzeichnet, dass** es sich bei dem Polyamin a) um ein Diamin handelt, welches aus den sekundär-primären, primär-primären oder sekundär-sekundären, vorzugsweise den sekundär-primären oder sekundär-sekundären Diaminen ausgewählt ist.

**11.** Oligomer nach Anspruch 10, **dadurch gekennzeichnet, dass** das Diamin aus den sekundär-primären Diaminen, vorzugsweise aus den folgenden, ausgewählt ist: N-Methyl-1,3-propandiamin, N-Methylethandiamin, N-Methyl-1,4-butandiamin, N-Methyl-1,5-pentandiamin.

**12.** Oligomer nach Anspruch 10, **dadurch gekennzeichnet, dass** das Diamin aus den sekundär-sekundären Diaminen, vorzugsweise aus den folgenden, ausgewählt ist: N-Methyl-N’methyl-1,3-propandiamin, N-Methyl-N’-methylethandiamin, N-Methyl-N’methyl-1,4-butandiamin, N-Methyl-N’methyl-1,5-pentandiamin.

**13.** Oligomer nach einem der Ansprüche 4 bis 7, **dadurch gekennzeichnet, dass** es sich bei dem Polyamin a) um ein Amin nach der Formel a2) gemäß der Begriffsbestimmung im Anspruch 1 handelt, wobei $n_2 = 1$ ist und $R_1$ und R’ für H stehen und es sich bei $R'_2$ et $R''_2$ um Alkylene handelt, welche vollkommen gleichartig oder verschiedenartig und des Typs $C_2$ à $C_6$ , insbesondere des Typs $C_2$ bis $C_4$ sind, wobei das Amin a2) insbesondere aus 3-(2-Aminoethyl)aminopropylamin oder bis-(3-Aminopropyl)amin ausgewählt ist.

**14.** Oligomer nach einem der Ansprüche 1 bis 7, **dadurch gekennzeichnet, dass** es sich bei dem Polyamin a) um ein Amin nach der Formel a2) gemäß der Begriffsbestimmung in Anspruch 1 handelt, wobei $n_2 = 2$ oder 3 ist und wobei $R_1$ und R’ für H stehen und wobei es sich bei $R'_2$ und $R''_2$ um vollkommen gleichartige Alkylene des Typs $C_2$ bis $C_4$ , insbesondere des Typs $C_2$ bis $C_3$ handelt, wobei das Amin a2) insbesondere aus Triethyltetramin oder Tetraethylenpentamin ausgewählt ist.

**15.** Oligomer nach einem der Ansprüche 1 bis 14, **dadurch gekennzeichnet, dass** es sich bei der Verbindung d) um ein multifunktionelles Acrylmonomer mit einem Funktionalisierungsgrad handelt, der mindestens 2 beträgt, insbesondere größer als 2 ist und so groß sein kann, dass p+1 gleich 6 ist, wobei es aus Folgendem ausgewählt ist:

d1) den Acrylatester von alkoxylierten oder nichtalkoxylierten Polyolen, vorzugsweise von Polyolen, die aus der folgenden Gruppe ausgewählt sind: Trimethylolpropan, Ditrimethylolpropan, Pentaerythrit, Dipentaerythrit, Diethylenglykol, Dipropylenglykol, Tripropylenglykol, Glycerin, Propylenglykol, Butandiol, Hexandiol oder
d2) den Epoxyacrylaten,
d3) den Aminoacrylaten.

**16.** Oligomer nach einem der Ansprüche 1 bis 14, **dadurch gekennzeichnet, dass** es sich bei der Verbindung d) um ein multifunktionelles Acryloligomer mit einem Funktionalisierungsgrad handelt, der mindestens 2 beträgt, insbesondere größer als 2 ist und so groß sein kann, dass p+1 gleich 12 ist, wobei es aus Folgendem ausgewählt ist:

d4) acrylmodifizierten Acryloligomeren, wobei es sich um ein Copolymer von acrylmodifiziertem Glycidylmethacrylat oder acrylmodifiziertem Styrololigomer handeln kann, bei welchem es sich um ein Copolymer von Styrol mit Maleinsäureanhydrid oder Acrylsäure handeln kann, das mit Hydroxyethylacrylat acrylmodifiziert ist,
d5) acrylmodifizierten hydroxylierten Oligomeren, insbesondere acrylmodifizierten hydroxylierten Polydienen, vorzugsweise acrylmodifiziertem hydroxyliertem Polybutadien und stärker bevorzugt acrylmodifiziertem hydriertem hydroxyliertem Polybutadien
d6) acrylmodifiziertem epoxidmodifiziertem Öl
d7) acrylmodifiziertem epoxidmodifiziertem Oligodien
d8) Oligoetheracrylaten
d9) Oligoesteracrylaten

d10) Oligoaminoacrylaten.

**17.** Oligomer nach einem der Ansprüche 1 bis 16, **dadurch gekennzeichnet, dass** der Funktionalisierungsgrad f bezüglich der Acrylat- und/oder Methacrylatgruppen im Bereich von 1 bis 30 liegt, wobei im Fall einer Monofunktionalität f = 1 ist, wobei es sich vorzugsweise um ein multifunktionelles Oligomer mit einem Funktionalisierungsgrad f handelt, der im Bereich von 2 bis 20, insbesondere von 3 bis 20, liegen kann.

**18.** Oligomer nach einem der Ansprüche 1 bis 17, **dadurch gekennzeichnet, dass** p im Bereich von 1 bis 11, vorzugsweise von 2 bis 11, stärker bevorzugt von 2 bis 5 liegen kann, wobei er noch stärker bevorzugt mehr als 2 und bis zu 5 betragen kann.

**19.** Oligomer nach einem der Ansprüche 1 bis 18, **dadurch gekennzeichnet, dass** m = 2 ist und dass p im Bereich von 2 bis 11 liegen kann und p vorzugsweise im Bereich von 3 bis 5 liegen kann.

**20.** Oligomer nach einem der Ansprüche 1 bis 19, **dadurch gekennzeichnet, dass** die Verbindung d) gegenüber den Amingruppen -NH- des Zwischenprodukts c) im molaren Überschuss vorliegt, wobei weiterhin die restliche Verbindung d) in den Oligomer als reaktionsfähiges Verdünnungsmittel vorliegt.

**21.** Verfahren zur Herstellung eines Oligomers gemäß der Begriffsbestimmung in einem der Ansprüche 1 bis 20, **dadurch gekennzeichnet, dass** es die folgenden Schritte umfasst:

i) Reaktion zwischen dem Polyamin a) gemäß der Begriffsbestimmung in einem der Ansprüche 1 bis 4, 7 bis 14 und 19 sowie der Carbonatverbindung b), welche mit m zyklischen Carbonaten versehen ist, gemäß der Begriffsbestimmung in einem der Ansprüche 1, 2, 5, 8, 9 und 19, wobei das Polyamin gegenüber b) im stöchiometrischen Überschuss vorliegt und allmählich dem Carbonat b) zugesetzt wird, welches zu Beginn im Reaktor vorliegt, und wobei die Reaktion ein Zwischenprodukt c) ergibt, welches mit m Urethangruppen und mit m Hydroxylgruppen in alpha- oder beta-Stellung zu dem Urethan versehen ist und welches mit verbleibenden reaktionsfähigen Amingruppen -NH- versehen ist,
ii) Reaktion zur Modifizierung der Verbinduing c), indem jede der verbleibenden reaktionsfähigen Amingruppen des Produkts c) an eine Acrylatgruppe einer Verbindung d) angelagert wird, welche über die Acrylatgruppe hinaus mit p zusätzlichen Acrylat- und/oder Methacrylatgruppen gemäß der Begriffsbestimmung in einem der Ansprüche 1, 6, 15 und 16 versehen ist, wobei auf diese Weise jede der verbleibenden reaktionsfähigen Amingruppen -NH- des Produkts c) in eine Kohlenstoff-Stickstoff-Bindung umgewandelt wird, welche mit den Acrylat- und/oder Methacrylatgruppen versehen ist, und auf diese Weise das Urethanoligomer erhalten wird,
wobei

- das Polyamin a) mit n primären und/oder sekundären Amingruppen sowie möglicherweise mit mindestens einer tertiären Aminogruppe versehen ist, wobei n im Bereich von 2 bis 5, vorzugsweise von 2 bis 4 und stärker bevorzugt von 2 bis 3 liegt, wobei es mit noch größerem Vorzug gleich 2 ist,
- das Carbonat b) mit m Carbonatgruppen versehen ist, wobei m im Bereich von 1 bis 2 liegt, wobei es sich bei dem Ring der zyklischen Carbonatgruppen vorzugsweise um einen Ring mit 5 oder mit 6 Atomen handelt,
- das Zwischenprodukt c) mit m Urethangruppen und mit m Hydroxylgruppen in alpha- oder beta-Stellung zu dem Urethan und mit m*(n-1) bis m*(2n-2) verbleibenden reaktionsfähigen Amingruppen -NH- versehen ist,
- die Verbindung d) über die Acrylatgruppe, welche mit den verbleibenden reaktionsfähigen Amingruppen -NH- reagiert, hinaus mit p Acrylat- und/oder Methacrylatgruppen versehen ist, wobei p mindestens gleich 1 ist und vorzugsweise mindestens gleich 2 ist, und
- die Anzahl der verbleibenden reaktionsfähigen Amingruppen -NH- höchstens gleich der Molanzahl der Verbindung d) ist,
- jede der Kohlenstoff-Stickstoff-Bindungen nach ihrer Bildung mit p Acrylat- und/oder Methacrylatgruppen versehen ist und das Urethanoligomer einen Funktionalisierungsgrad bezüglich Acrylaten und/oder Methacrylaten hat, welcher im Bereich von m*p(n-1) bis m*p(2n-2) liegt.

**22.** Vernetzbare Zusammensetzung, **dadurch gekennzeichnet, dass** sie als Bindemittel mindestens ein Oligomer umfasst, welches der Begriffsbestimmung in einem der Ansprüche 1 bis 20 entspricht oder mittels eines Verfahrens gemäß der Begriffsbestimmung im Anspruch 21 hergestellt wurde.

**23.** Vernetzbare Zusammensetzung nach Anspruch 22, **dadurch gekennzeichnet, dass** sie durch Strahlungseinwirkung und/oder auf dem thermischen oder bei niedrigen Temperaturen durchgeführten Peroxidweg und/oder auf dem Michael-Additionsweg im Falle von Oligomeren, welche mit mindestens zwei funktionellen Acrylatgruppen versehen sind, und/oder auf einem anderen dualen Weg vernetzt werden kann, bei welchem mindestens zwei der genannten Wege zum Einsatz kommen und/oder bei welchem die Gesamtheit oder ein Teil der m verbleibenden Hydroxylgruppen des Oligomers zum Einsatz kommen, indem letztere mit einem Vernetzungsmittel umgesetzt werden, das mit den Hydroxylgruppen reaktionsfähig ist.

**24.** Vernetzbare Zusammensetzung nach Anspruch 22 oder 23, **dadurch gekennzeichnet, dass** es sich um eine Zusammensetzung aus Beschichtungen, die vorzugsweise zu den Beschichtungen für Anstrichmittel, Klarlacke und Druckfarben gehören, um eine Formgebungszusammensetzung, eine Zusammensetzung aus chemischen Abdichtungs- oder Versiegelungsmitteln, eine Zusammensetzung aus Klebstoffen, eine Zusammensetzung für Systeme zur schichtweisen 3D-Herstellung von Gegenständen, eine Zusammensetzung für 3D-Drucksysteme, eine Betonzusammensetzung oder um eine Verbundstoffzusammensetzung handelt.

**25.** Verwendung eines Oligomers, welches der Begriffsbestimmung nach einem der Ansprüche 1 bis 20 entspricht oder mittels des Verfahrens gemäß der Begriffsbestimmung im Anspruch 21 erhalten wurde, als reaktionsfähiges Bindemittel in Zusammensetzungen aus Beschichtungen, insbesondere Anstrichmitteln, Klarlacken und Druckfarben, aus Klebstoffen, zur Formgebung, aus chemischen Abdichtungs-, Versiegelungsmitteln, für Systeme zur schichtweisen 3D-Herstellung von Gegenständen, für 3D-Drucksysteme, aus Beton oder aus Verbundwerkstoff.

**26.** Fertigprodukt, **dadurch gekennzeichnet, dass** es entsteht, indem mindestens ein Oligomer verwendet wird, welches der Begriffsbestimmung nach einem der Ansprüche 1 bis 20 entspricht oder mittels des Verfahrens gemäß der Begriffsbestimmung im Anspruch 21 erhalten wurde, wobei das Produkt vorzugsweise aus den Beschichtungen, insbesondere den Beschichtungen für Anstrichmittel, Klarlacke und Druckfarben, den Produkten ausgewählt ist, welche zu den Fügemitteln mit Haftwirkung, den Formteilen, den 3D-Gegenständen, die schichtweise erhalten wurden, den 3D-Druckerzeugnissen, den Dichtungen, den chemischen Versiegelungsmitteln, Fertigbeton oder Gegenständen aus Verbundwerkstoff gehören.

## Claims

**1.** Urethane oligomer which is monofunctional or multifunctional in acrylates and/or methacrylates, preferably acrylates, which can be obtained from the reaction of a specific polyamine a) with a cyclic carbonate compound b) bearing m cyclic carbonate groups, said polyamine being in stochiometric excess with respect to b) and said reaction giving an intermediate product c) bearing m formed urethane groups, which groups bear residual reactive amine -NH- groups, and subsequently by an addition reaction of each of said residual reactive amine groups of said product c) with an acrylate group of a compound d) bearing, in addition to said acrylate group, p additional acrylate and/or methacrylate groups, with each residual reactive amine -NH- group of said product c) being thus converted into a carbon-nitrogen bond bearing said acrylate and/or methacrylate groups, and thus production of said urethane oligomer, with:

- said polyamine a) bearing n primary and/or secondary amine groups and optionally at least one tertiary amine group, with n ranging from 2 to 5, preferably from 2 to 4 and more preferentially from 2 to 3, more preferentially still of 2,
- said carbonate b) bearing m cyclic carbonate groups with m ranging from 1 to 2, the ring of said cyclic carbonate groups preferably being a ring having 5 or 6 atoms,
- said intermediate product c) bearing m urethane groups and m OH groups in the alpha or beta position with respect to said urethane group and c) bearing from $m^*(n-1)$ to $m^*(2n-2)$ residual reactive amine -NH- groups,
- said compound d) bearing p acrylate and/or methacrylate groups in addition to said acrylate group which reacts with one of said residual reactive amine -NH- groups, with p being at least equal to 1 and preferably at least equal to 2, and
- the number of said residual reactive amine -NH- groups of said product c) being less than or equal to the number of moles of said compound d),
- each carbon-nitrogen bond formed bearing p acrylate and/or methacrylate groups and said urethane oligomer bearing m urethane groups and m hydroxyl groups in the alpha or beta position with respect to said urethane and having a functionality in acrylates and/or methacrylates ranging from $m^*p(n-1)$ to $m^*p(2n-2)$

said polyamine a) being chosen from polyamines of following formulae a1) and a2):

a1) $R_1$-NH-$R_2$- (NH-R) (NH-R')$_{n1}$, with $n_1$ being equal to 3, 2, 1 or 0
a2) $R_1$-NH-(R' $_2$NH) $_{n2}$-R"$_2$-NH-R' with $n_2$ being equal to 3, 2 or 1,

with $R_1$ and R and R' being independently chosen from: H, alkyl, preferably $C_1$-$C_3$ alkyl, said alkyl optionally being alkoxylated or a cycloalkyl, with it being possible for $R_1$ to be identical to or different from R and R' and with it being possible for R and R' to be identical or different and in particular with R and/or R' being different from H,
$R_2$ being alkylene or cycloalkylene (comprising an aliphatic $C_6$ ring) or aralkylene of valency n ranging from 2 to 5, with it being possible for $R_2$ to bear or comprise at least one tertiary amine group,
and
it being possible for R'$_2$ and R"$_2$ to be identical or different and to be chosen from alkylenes, preferably $C_2$ to $C_8$ alkylenes and more preferably $C_2$ to $C_6$ alkylenes, and, if R"$_2$ is different from R'$_2$, in this case it being possible for R"$_2$ to bear or comprise at least one tertiary amine group.

2. Oligomer according to Claim 1, **characterized in that** said n primary and/or secondary amine groups of said polyamine a) can be borne by a radical A of valency n, which radical A can be or comprise an aliphatic, cycloaliphatic or aromatic structure with, in the latter case, said n amine groups being borne by at least one alkylene group attached to the aromatic ring, and the rings of said m cyclic carbonate groups of said carbonate b) have 5 or 6 atoms and are borne by a radical $R_3$ of valency m, with $R_3$ being a hydrocarbon radical of aliphatic, cycloaliphatic or aromatic structure and optionally hydroxylated or $R_3$ can be a simple hydrogen when m = 1; optionally, the carbonate ring can be substituted by an optionally hydroxylated hydrocarbon substituent $R_4$, it being possible for $R_4$ to be of aliphatic, cycloaliphatic or aromatic structure identical to or different from that of $R_3$ and it optionally being possible for $R_4$ to form a ring attached to the carbonate ring by two common carbon atoms.

3. Oligomer according to Claim 1 or 2, **characterized in that** said polyamine a) is chosen from:

- a $C_2$ to $C_{54}$ alkylenepolyamine, said alkylene being a linear or branched $C_2$ to $C_{54}$ alkylene and bearing said n primary and/or secondary amine groups,
- a polyamine comprising at least two repeat units from among: $C_2$ to $C_4$ alkyleneimine, in particular ethyleneimine or propyleneimine, preferably ethyleneimine, said polyamine a) being in this case an oligoalkyleneiminepolyamine, in particular a polyethyleneiminepolyamine, including of dendritic structure,
- a polyamine comprising one or more units from among: ether, ester-amide or amide, with, in the case of the presence of several units, said polyamine a) being chosen from: oligoether polyamines, oligoester amide-polyamines or multiester amide-polyamines.

4. Oligomer according to one of Claims 1 to 3, **characterized in that** said polyamine a) is chosen from polyamines of formula a1) with $n_1$ being equal to 2, 1 or 0 or from polyamines of formula a2) with $R_1$ and R and R' being independently chosen from $C_1$-$C_3$ alkyl, said alkyl optionally being alkoxylated or a cycloalkyl, with it being possible for $R_1$ to be identical to or different from R and R' and with it being possible for R and R' to be identical or different and with R and/or R' being different from H.

5. Oligomer according to one of Claims 1 to 4, **characterized in that** said carbonate b) is a complete or partial ester of a monomeric or oligomeric polyacid, such as an oligoester polyacid, with a hydroxylated carbonate, preferably glycerol carbonate, or b) is a complete or partial ether of a monomeric or oligomeric polyol, such as oligoester or oligoether polyol, with a hydroxylated carbonate, preferably glycerol carbonate, or b) is a complete or partial ether of a phenolic derivative, including bisphenol A or dihydroxyphenylene, with a hydroxylated carbonate, preferably glycerol carbonate, or b) is an ether of allyl or vinyl alcohol with a hydroxylated carbonate, preferably glycerol carbonate or a methacrylate of a hydroxylated carbonate, preferably glycerol carbonate, or b) is a carbonate obtained by addition of $CO_2$ to a precursor monomeric or oligomeric epoxidized compound or b) is the product of the reaction of dimethyl carbonate with an aliphatic or cycloaliphatic polyol with a functionality of at least 2, preferably ranging from 2 to 6.

6. Oligomer according to one of Claims 1 to 5, **characterized in that** said compound d) is represented overall by $R_5$-($O_2$CCH=$CH_2$) (-$O_2$CC ($R_6$) =$CH_2$)$_p$ with $R_5$ being a hydrocarbon residue of valency p+1 and $R_6$ is H or methyl for a portion or all of the p groups, and preferably $R_5$ is a polyol residue chosen from optionally alkoxylated and/or substituted alkylene polyol, optionally substituted cycloalkylene polyol or aralkylene polyol optionally alkoxylated and/or substituted on the aromatic ring or polyether polyol or polyester polyol or $R_5$ is a residue of a multifunctional epoxy acrylate compound bearing an acrylate group and p additional acrylate and/or methacrylate groups.

7. Oligomer according to one of Claims 1 to 6, **characterized in that** said polyamine a) is a diamine.

8. Oligomer according to one of Claims 4 to 7, **characterized in that** the carbonate ring of said carbonate b) has 5 atoms, that said polyamine a) is a secondary-primary diamine according to formula a1) as defined according to Claim 1, with $n_1 = 0$ and R = H, and that said oligomer comprises at least one oligomer of following general formula (I):

$$[(CH_2=CH(R_6)-CO_2)_p-R_5-O_2C-CH_2-CH_2-N(R_1)-R_2-NH-CO_2-C(R_4)-CH(OH)-]_mR_3 \quad (I)$$

with

$R_1$ being as defined according to Claim 1,
$R_2$ being as defined according to Claim 1,
$R_3$ being as defined according to Claim 2,
$R_4$ being as defined according to Claim 2,
$R_5$ being as defined according to Claim 7,
$R_6$ being H or methyl or H and methyl if p is other than 1.

9. Oligomer according to one of Claims 1 to 7, **characterized in that** said carbonate ring of said carbonate b) has 6 atoms, that said polyamine a) is a secondary-primary diamine according to formula a1) as defined in Claim 1, with $n_1 = 0$ and R = H, and that said oligomer comprises at least one oligomer of following general formula (II):

$$[(CH_2=CH(R_6)-CO_2)_p-R_5-O_2C-CH_2-CH_2-N(R_1)-R_2-NH-CO_2-C(R_4)-CH_2CH(OH)-]_mR_3 \quad (II)$$

with $R_1$, $R_2$, $R_3$, $R_4$, $R_5$ and $R_6$ being as defined according to Claim 8.

10. Oligomer according to one of Claims 1 to 7, **characterized in that** said polyamine a) is a diamine selected from secondary-primary, primary-primary or secondary-secondary diamines, preferably secondary-primary or secondary-secondary diamines.

11. Oligomer according to Claim 10, **characterized in that** said diamine is selected from secondary-primary diamines, preferably from: N-methyl-1,3-propanediamine, N-methylethanediamine, N-methyl-1,4-butanediamine or N-methyl-1,5-pentanediamine.

12. Oligomer according to Claim 10, **characterized in that** said diamine is selected from secondary-secondary diamines, preferably from: N-methyl-N'-methyl-1,3-propanediamine, N-methyl-N'-methylethanediamine, N-methyl-N'-methyl-1,4-butanediamine or N-methyl-N'-methyl-1,5-pentanediamine.

13. Oligomer according to one of Claims 4 to 7, **characterized in that** said polyamine a) is an amine according to formula a2) as defined according to Claim 1, with $n_2 = 1$ and $R_1$ and R' being H and $R'_2$ and $R''_2$ being identical or different $C_2$ to $C_6$ alkylenes, in particular $C_2$ to $C_4$ alkylenes, said amine a2) being more particularly selected from 3-[(2-aminoethyl)amino]propylamine or bis(3-aminopropyl)amine.

14. Oligomer according to one of Claims 1 to 7, **characterized in that** said polyamine a) is an amine according to formula a2) as defined according to Claim 1, with $n_2 = 2$ or 3 and $R_1$ and R' being H and $R'_2$ and $R''_2$ being identical $C_2$ to $C_4$ alkylenes, in particular $C_2$ to $C_3$ alkylenes, said amine a2) being more particularly selected from triethylenetetramine or tetraethylenepentamine.

15. Oligomer according to one of Claims 1 to 14, **characterized in that** said compound d) is a multifunctional acrylic monomer having a functionality of at least 2, in particular greater than 2 and ranging up to p+1 equal to 6 and selected from:

d1) acrylate esters of alkoxylated or nonalkoxylated polyols, preferably polyols chosen from the group: trimethylolpropane, ditrimethylolpropane, pentaerythritol, dipentaerythritol, diethylene glycol, dipropylene glycol, tripropylene glycol, glycerol, propylene glycol, butanediol and hexanediol, or
d2) epoxy acrylates,
d3) aminoacrylates.

**16.** Oligomer according to one of Claims 1 to 14, **characterized in that** said compound d) is a multifunctional acrylic oligomer having a functionality of at least 2, in particular greater than 2 and ranging up to p+1 equal to 12 and selected from:

d4) acrylated acrylic oligomer which can be an acrylated glycidyl methacrylate copolymer or acrylated styrene oligomer which can be a copolymer of styrene with maleic anhydride or acrylic acid which is acrylated by hydroxyethyl acrylate,
d5) acrylated hydroxylated oligomer, in particular from acrylated hydroxylated polydienes, preferably acrylated hydroxylated polybutadiene and more preferentially acrylated hydrogenated hydroxylated polybutadiene,
d6) acrylated epoxidized oil,
d7) acrylated epoxidized oligodiene,
d8) oligoether acrylates,
d9) oligoester acrylates,
d10) oligoaminoacrylates.

**17.** Oligomer according to one of Claims 1 to 16, **characterized in that** the functionality f in acrylate and/or methacrylate groups varies from 1 to 30, with f = 1 if monofunctional, preferably multifunctional oligomer with a functionality f ranging from 2 to 20, more particularly from 3 to 20.

**18.** Oligomer according to one of Claims 1 to 17, **characterized in that** p varies from 1 to 11, preferably from 2 to 11, more preferentially from 2 to 5, more preferentially still being greater than 2 and ranging up to 5.

**19.** Oligomer according to one of Claims 1 to 18, **characterized in that** m = 2 and that p varies from 2 to 11 and preferably p varies from 3 to 5.

**20.** Oligomer according to one of Claims 1 to 19, **characterized in that** said compound d) is in molar excess with respect to the amine -NH- groups of said intermediate product c) and with said residual compound d) being present in said oligomer as reactive diluent.

**21.** Process for the preparation of an oligomer as defined according to one of Claims 1 to 20, **characterized in that** it comprises the following steps:

i) reaction between said polyamine a) as defined according to one of Claims 1 to 4, 7 to 14 and 19 and said carbonate compound b) bearing m cyclic carbonates as defined according to one of Claims 1, 2, 5, 8, 9 and 19, said polyamine being in stochiometric excess with respect to b) and being gradually added to said carbonate b) present at the start in the reactor and said reaction giving an intermediate product c) bearing m urethane groups and m hydroxyl groups in the alpha or beta position with respect to said urethane and bearing the residual reactive amine -NH- groups,
ii) reaction for the modification of said compound c), by addition of each of said residual reactive amine groups of said product c) to an acrylate group of a compound d) bearing, in addition to said acrylate group, p additional acrylate and/or methacrylate groups as defined according to one of Claims 1, 6, 15 and 16, with each residual reactive amine -NH- group of said product c) being thus converted into a carbon-nitrogen bond bearing said acrylate and/or methacrylate groups, and thus production of said urethane oligomer,

with

- said polyamine a) bearing n primary and/or secondary amine groups and optionally at least one tertiary amine group, with n ranging from 2 to 5, preferably from 2 to 4 and more preferentially from 2 to 3, more preferentially still of 2,
- said carbonate b) bearing m carbonate groups with m ranging from 1 to 2, the ring of said cyclic carbonate groups preferably being a ring having 5 or 6 atoms,
- said intermediate product c) bearing m urethane groups and m hydroxyl groups in the alpha or beta position with respect to said urethane and from m*(n-1) to m*(2n-2) residual reactive amine -NH- groups,
- said compound d) bearing p acrylate and/or methacrylate groups in addition to said acrylate group which reacts with one of said residual reactive amine -NH- groups, with p being at least equal to 1 and preferably at least equal to 2, and
- the number of said residual reactive amine -NH- groups being less than or equal to the number of moles of said compound d),

- each carbon-nitrogen bond formed bearing p acrylate and/or methacrylate groups and said urethane oligomer having a functionality of acrylates and/or methacrylates ranging from m*p(n-1) to m*p(2n-2).

**22.** Crosslinkable composition, **characterized in that** it comprises, as binder, at least one oligomer as defined according to one of Claims 1 to 20 or as obtained by a process as defined according to Claim 21.

**23.** Crosslinkable composition according to Claim 22, **characterized in that** it can be crosslinked by the radiation route and/or by the thermal or low-temperature peroxide route and/or by the Michael-type addition route, in the case of oligomers bearing at least two acrylate functional groups, and/or by another dual route involving at least two of the routes mentioned and/or involving all or a portion of the m residual hydroxyl groups of said oligomer, by reaction of these residual hydroxyl groups with a crosslinking agent which reacts with said hydroxyls.

**24.** Crosslinkable composition according to Claim 22 or 23, **characterized in that** it is a coating composition, preferably from coatings for paints, varnishes and inks, a moulding composition, a leaktightness agent or chemical sealing composition, an adhesive composition, a composition for systems for the layer-by-layer manufacture of 3D objects, a composition for 3D printing systems, a concrete composition or a composite composition.

**25.** Use of an oligomer as defined according to one of Claims 1 to 20 or obtained by the process as defined according to Claim 21 as reactive binder in coating compositions, in particular paints, varnishes and inks, adhesive compositions, moulding compositions, leaktightness agent compositions, chemical sealing compositions, compositions for systems for the layer-by-layer manufacture of 3D objects, compositions for 3D printing systems, concrete compositions or composite compositions.

**26.** Finished product, **characterized in that** it results from the use of at least one oligomer as defined according to one of Claims 1 to 20 or obtained by the process as defined according to Claim 21, preferably a product chosen from coatings, in particular coatings for paints, varnishes and inks, or a product from adhesive seals, moulded parts, 3D objects obtained layer-by-layer, 3D printings, leaktight seals, chemical sealing, finished concrete or composite articles.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20130004677 A1
- US 2004254292 A1
- US 2004192803 A1


### Littérature non-brevet citée dans la description


- *Macromolecules,* 1976, vol. 9, 199-211